(19) Europäisches Patentamt
European Patent Office
Office européen des brevets

(11) EP 3 431 612 A1

(12) EUROPEAN PATENT APPLICATION

(43) Date of publication:
**23.01.2019 Bulletin 2019/04**

(51) Int Cl.:
*C12Q 1/68* (2018.01) *G06F 19/22* (2019.01)

(21) Application number: **17305968.4**

(22) Date of filing: **20.07.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **INSERM (Institut National de la Santé et de la Recherche Médicale)**
  **75013 Paris (FR)**
- **CENTRE NATIONAL DE LA RECHERCHE SCIENTIFIQUE**
  **75016 Paris (FR)**
- **Université de Paris 5 René Descartes**
  **75006 Paris (FR)**
- **Assistance Publique-Hôpitaux de Paris (AP-HP)**
  **75004 Paris (FR)**

(72) Inventors:
- **LETOURNEUR, Franck**
  **78700 CONFLANS-SAINTE-HONORINE (FR)**
- **ASSIÉ, Guillaume**
  **92190 MEUDON (FR)**
- **BERTHERAT, Jérôme**
  **75006 PARIS (FR)**

(74) Representative: **Novagraaf Technologies**
**Bâtiment O2**
**2, rue Sarah Bernhardt**
**CS90017**
**92665 Asnières-sur-Seine Cedex (FR)**

# (54) METHOD FOR MOLECULAR TYPING OF TUMORS IN A SINGLE TARGETED NEXT GENERATION SEQUENCING EXPERIMENT

(57) The present invention concerns a method of analyzing a cancer of a patient, in particular an adrenocortical carcinoma, on the basis of determining (i) chromosomal alterations identified specifically for said cancer and (ii) DNA methylation status of genes of said chromosome regions, in a single targeted NGS.

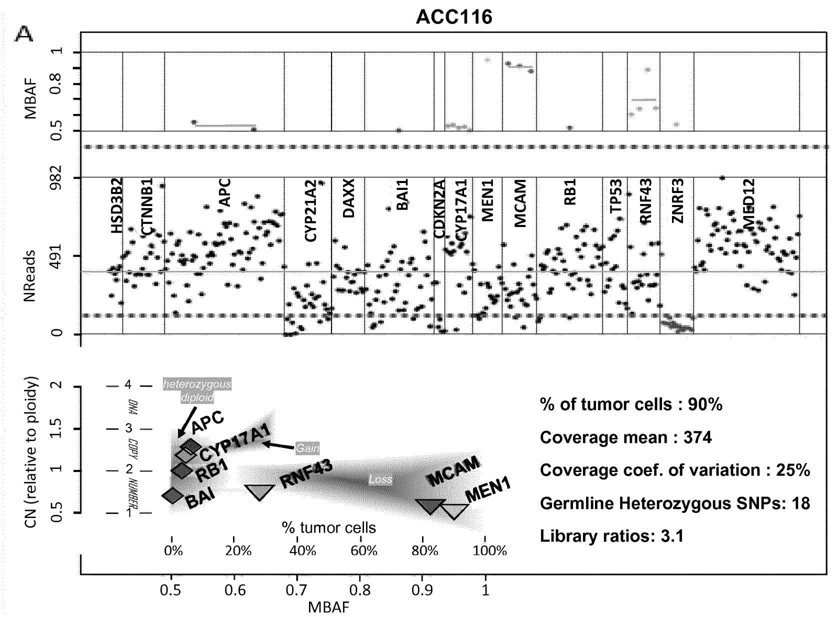


FIGURE 3

EP 3 431 612 A1

## Description

### Field of the invention

The present invention concerns a new method of analyzing a cancer of a patient by detecting gene mutations, chromosomal alterations and DNA methylation status in a targeted Next generation sequencing (NGS) experiment.

The present invention applies in the medical field, particularly to improve tumor classification for each patient.

In the description below, the references between square brackets ([ ]) refer to the list of references presented at the end of the text.

### Background of the invention

Genomics performs high throughput detection of molecular variations, at the gene expression level (by transcriptome [1-3] and miRnome experiments [1,4]) which, for example, helped to distinguish tumors involving good or poor prognosis by identifying different molecular types, as for example for adrenocortical carcinoma [1], at the genomic DNA sequence level (by targeted/exome/whole genome sequencing [3]), at the chromosomal structure level (by SNP and CGH arrays, and by exome/whole genome sequencing [5-7]), and at the genomic DNA methylation level (by methylation arrays [8,9] or by DNA sequencing, after either treatment of DNA by bisulfite [9] or methyl cytosine immunoprecipitation) [10].

Tumor tissues have been extensively studied by pangenomic approaches. Indeed, an ever increasing number of tumor types have now been extensively screened by these techniques, with the global aim of identifying molecular subtypes and unraveling the molecular mechanisms of tumorigenesis [3,7,11-15]. Some of the molecular features thus identified may have important implications for patient's care. These include strong diagnostic and prognostic markers [16,17], and molecular signatures orienting towards specific treatments including targeted therapies [17,18]. In the post-genomic era, it is therefore of major importance to be able to translate the massive pangenomic features into targeted molecular measurements, compatible with clinical routine [19-21]. Thus pangenomic studies identified distinct molecular classes for many cancers, with major clinical applications. However, routine use requires conversion to cost effective assays.

Targeted next generation sequencing (NGS) is a powerful, robust and cost-effective technology in clinical practice [22,23]. Several applications are now emerging, including rapid screening of multiple genes in genetic diseases, identification of specific somatic mutations in different cancer types [24,25], and characterization of viruses and bacteria [26-28]. For these reasons targeted NGS is rapidly spreading aside clinical departments.

All these applications are primarily based on ability of targeted NGS to identify DNA sequence variations, namely base substitutions and indels [6]. Unconventional uses of targeted NGS are emerging : several methods for detecting copy number based on NGS have been reported. Some show ability to detect homozygous deletions [29,30]. Other methods propose to identify variations of one DNA copy and loss of heterozygosity (LOH) [5,6,29-33], using approaches similar to methods developed for SNP arrays [34-36]. These methods have been developed for analyzing large genomic regions - e.g. whole genome sequencing (WGS) data, or whole exome sequencing (WES) - much larger than common targeted NGS panels used in clinical oncogenetics. Therefore, these methods are not suitable, and nor optimized for small targeted NGS panels.

Thus there is still a need for a method for the molecular typing of tumors compatible with clinical routine, at a limited cost increase. Such routine molecular typing might be of a particular advantage in cancer diagnostic and/or prognostic, as well as in the choice of a pertinent treatment thereof.

### Detailed description of the invention

Considering the wide use of small targeted Next-Generation Sequencing (NGS) panels, the aim of the present invention was to assess whether these panels can serve for detecting specific gene mutations, calling chromosomal alterations and DNA methylation status. Those three combined analyses allowing classification of tumors according molecular typing.

The present invention especially relates to a NGS method for classifying cancerous tumors comprising using a set of genes of chromosome regions specifically identified in the art for said cancer, for which a search for mutations, an analysis of chromosomal abnormalities as well as an analysis of targeted hypermethylated regions is performed in only one run. This method thus allows analysis, in a single NGS experiment, of various events useful for typing tumors: mutations, chromosomal alterations (loss of heterozygosity, alterations, duplication, deletion) and methylation.

As shown in the experimental section, Inventors have been able to characterize chromosomal alterations in 449 tumors from 42 different cancer types through the NGS experiment of the invention, which, as shown for adrenocortical cancer, when comprising analyses of mutations and the assessment of DNA methylation led to the precise molecular typing of tumors. Hence, this invention is particularly suitable, in clinical routine, for the molecular typing and classification of tumor of each patient. Furthermore, in the same NGS experiment, a second sequencing library is added to include DNA methylation status which is of a particular advantage for oncogenetics analyses.

Accordingly, in an embodiment, the invention relates to a Next-Generation DNA Sequencing (NGS) method of analysing a cancer of a patient comprising the detection, in a sample of said patient, of:

- at least one characteristic alteration of chromosome regions identified for said cancer from a set of genes from these regions,
- specific gene mutations or at least one characteristic pattern of mutations in a set of genes identified in said cancer, and
- at least one characteristic pattern of DNA methylation status of chromosome regions identified as having an altered methylation status in said cancer,

all these three detection steps being implemented in a single NGS experiment.

As explained above, in a further embodiment, the detection step of detecting at least one characteristic alteration of chromosome regions of said method comprises identifying homozygous deletions and/or loss of heterozygosity (LOH) within the set of genes of said chromosome regions identified for said cancer.

Advantageously, in the method according to the invention, the detection step of at least one characteristic alteration of chromosome regions further comprises analysing at least 5 SNPs per chromosome arm of interest for searching heterozygous deletions or LOH (Loss of Heterozygosity), said at least 5 SNPs being known to be highly heterozygous in the general population. Inventors have indeed identified that detecting loss of one DNA copy using the method of the invention implies providing allelic ratios of heterozygous SNPs on each chromosome of interest. In another particular embodiment, said at least 5 SNPs are sequenced from patient leucocytes in addition to tumor. In a more particular embodiment, said at least 5 SNPs are sequenced from tumor only.

In a particular embodiment, the step of detecting at least one specific pattern of DNA methylation status is carried out onto bisulfite-treated DNA. In a more particular embodiment the analysis of the methylation status is implemented on CpG islands known as having an altered methylation status in said cancer. In a more particular embodiment the step of detecting at least one specific pattern of DNA methylation status is implemented on a subset of CpG islands, identified as sufficient for the cancer analysis of said patient.

Inventors have furthermore identified a method for increasing of more than 5 times the alignment efficiency over commonly used methylated sequencing methods. Therefore, methylation status analysis is advantageously operated (i) after a step of replacing the stretches of identical bases by only one corresponding base, except around the CpGs, the dinucleotides CG, TG and CA being excluded from this compression and (ii) with the alignment over the reference sequence restricted to the use of 3' primers end.

The methods of the invention are therefore suitable for detecting a mutation in a tumor suppressor gene, knowing the status of the other allele and the proportion of cells harboring the mutation. This is of particular interest for targeted therapies, as it is not yet currently assessed whether all tumor cells harbor a targeted mutation, or whether only a sub-clonal population will be targeted. Furthermore, the methods of the invention are also efficient in detecting homozygous deletions, high level amplifications which are common ways for inactivating tumor suppressor genes or activating oncogenes respectively, or even only one gain or loss of DNA copy. The methods of the invention also comprise a step of analysis DNA methylation status. DNA methylation status is also important for prognosis and potentially for treatment orientation ; indeed CpG island hypermethylation is a well-known mechanism of tumor suppressor. Then, beside calling mutations, using the method of the invention allows, in a single analysis, to detect the major determinants of molecular typing of tumors.

This is of particular advantage over the pangenomic studies which are not suitable for diagnosis or prognosis in clinical routine. Accordingly, the use of the above methods for assigning a patient to a specific group of patients corresponding to a specific molecular type of tumor is an object of the invention.

In a particular embodiment, said group of patients and/or the corresponding molecular type of tumor is indicative of the reponse of the disease to a treatment, and/or of the survival of the patient who is assigned to this particular group and/or molecular type of tumor.

In another particular embodiment the methods of the invention are used for stratifiying patients during clinical trial and/or for identifying molecular type of tumors that are indicative of a response to a treatment or allow to classify patients as a function of survival time expectancy. Consequently, in a more particular embodiment, the invention also relate a method of adapting the treatment of a cancer of a patient comprising the implementation of the steps of the targeted NGS method of the invention and a step of chosing the best therapeutic option for said patient as a fonction of the molecular type of tumor identified thereby for said patient. More particularly, "adapting the treatment of cancer" or "chosing the best therapeutic option" can comprise determining wether or not the patient is a responder to a treatment and, in a particular embodiment, thereby avoiding the administration of a useless treatment. It can comprise also chosing a targeted therapy known to be effective for the molecular type of cancer identified.

In the frame of the invention, "sample of a patient" comprises any tumor biopsy sample as incisional biopsy, excisional biopsy, or needle biopsy. "Sample of a patient" comprises also any autopsy samples, frozen samples dedicated to histologic analyses, fixed or wax embedded sample. As used herein the terms "sample of a patient" are preferably a tumor biopsy sample, but it can also be metastasis biopsy, or lymph node biopsy from a subject suffering or suspected to suffer from cancer or of cancer relapse. "Sample of a patient" can also comprise cells or cell lines or organoids or patient-derived xenografts (PDX) derived from patient tumor samples.

As shown in the experimental section, methods of the invention are suitable for detecting chromosomal alterations in any type of cancer. Indeed methods of the invention have been validated for detecting chromosomal alterations in 449 tumors from 42 different cancer types (Table 1), beside adrenocortical carcinoma. In a particular embodiment, methods of analysing cancer of a patient of the invention are implemented for a cancer selected from breast cancer, colorectal cancer, ovarian cancer, lung cancer, pancreatic cancer, sarcoma, urothelial cancer, head and neck squamous cell carcinoma (HNSCC), adenoma carcinoma with unknown primitive tumor (ACUP), endometrial cancer, cervical cancer, oesogastric cancer, adenoid cystic carcinoma (ACC), cholangiocarcinoma, neuroendocrine tumor, melanoma, anal squamous cell carcinoma (Anal SCC), kidney cancer, uveal melanoma, germline tumor, hepatocellular carcinoma (HCC), parotid cancer, thyroid cancer, undifferentiated nasopharyngeal cancer of the cavum (UCNT CAVUM), Merkel cell carcinoma, mesothelioma, penile squamous cell carcinoma, peritoneal cancer, chemodectoma, corticosurrenaloma, desmoid tumor, epithelioid hemangiocarcinoma, meningioma, midline carcinoma, mixopapillary ependymoma, non-adenoid cystic carcinoma (ACC) salivary gland tumor, ocular adenocarcinoma, pelvic squamous cell carcinoma (pelvic SCC), pleiomorphic carcinoma of the tongue, prostate adenocarcinoma, thymic cancer, or squamous cell carcinoma of the vulva.

Table 1

| **Validation samples** | | | |
|---|---|---|---|
| **Cancer** | **Number of Samples** | **Cancer** | **Number of sample** |
| Breast cancer | **70** | Parotid cancer | **3** |
| Colorectal cancer | **52** | Thyroid cancer | **3** |
| Ovarian cancer | **49** | UCNT CAVUM | **3** |
| Lung cancer | **41** | Merkel cell carcinoma | **2** |
| Pancreatic cancer | **36** | Mesothelioma | **2** |
| Sarcoma | **25** | Penile squamous cell carcinoma | **2** |
| Urothelial cancer | **17** | Peritoneal cancer | **2** |
| HNSCC | **16** | Chemodectoma | **1** |
| ACUP | **15** | Corticosurrenaloma | **1** |
| Endometrial cancer | **15** | Desmoid tumor | **1** |
| Cervical cancer | **12** | Epithelioid hemangiocarcinoma | **1** |
| Oesogastric cancer | **12** | Meningioma | **1** |
| ACC | **11** | Midine carcinoma | **1** |
| Cholangiocarcinoma | **10** | Mixopapillary ependymoma | **1** |
| Neuroendocrine tumor | **8** | Non-ACC salivary gland tumor | **1** |
| Melanoma | **6** | Ocular adenocarcinoma | **1** |
| Anal SCC | **5** | Pelvic SCC | **1** |
| Kidney cancer | **5** | Pleiomorphic carcinoma of the tongue | **1** |
| Uveal melanoma | **5** | Prostate adenocarcinoma | **1** |
| Germline tumor | **4** | Thymic cancer | **1** |
| HCC | **4** | Vulva SCC | **1** |

Regarding specifically adrenocortical carcinoma, the NGS method of the invention constitutes the first predictor of survival in patients only based on DNA analysis of tumors in a single NGS experiment; said method using a "set of targets" comprising at least one gene selected specifically among , *HSD3B2, CTNNB1*, *APC, CYP21A2, DAXX, BAI1*, *CDKN2A, CYP17A1, MEN1, MCAM, RB1, TP53, RNF43, ZNRF3, MED12,* CDK4, regarding mutations and homozygous deletions said at least one gene being distributed within about 10 regions identified as with frequent loss of heterozygosity (LOH), and about 4 CpG-rich regions selected from Cg07384961, Cg14021073, Cg21494776, Cg23130254, Cg20312228, Cg01635061, Cg27234090, Cg04582938, Cg06039392, Cg10167296, Cg10743104, Cg27425675, Cg16689634, Cg01120165, Cg15284635, wherein methylation events are known to occur (hypermethylated regions in aggressive cancers). Applying the set of targets as defined above, the NGS method of the invention allows to analyze various types of abnormalities of tumoral DNA, on targeted regions, and comprises:

1) Analysis of recurrent mutations in at least one gene selected from *HSD3B2, CTNNB1,APC, CYP21A2, DAXX, BAI1*, *CDKN2A, CYP17A1, MEN1, MCAM, RB1*, *TP53, RNF43, ZNRF3, MED12,* and CDK4.
2) Identification of SNP genotypes by measuring by allelic ratios and copy numbers in at least one of the above genes some genes, after capture of these genes by PCR, the amplicons coverage of the captured regions is evaluated. In comparison with the coverage of other genes, it is thus possible to identify specifically for a gene, a fall of the coverage for all amplicons of this gene. To optimize this quantification, capture of 5 to 10 SNPs with high heterozygosity is introduced on the same chromosome arm, so as to have an internal control for a better discrimination of homozygous deletions from heterozygous deletions.
3) Searching for loss of heterozygosity in at least one of the chromosomes arms selected from 1 p, 1 q, 2p, 2q, 9p, 11p, 11q, 17p, 18p, 18q, and 22q.
4) Analysis of the methylation status of 4 CpG-rich regions selected from Cg07384961, Cg14021073, Cg21494776, Cg23130254, Cg20312228, Cg01635061, Cg27234090, Cg04582938, Cg06039392, Cg10167296, Cg10743104, Cg27425675, Cg16689634, Cg01120165 and Cg15284635.

In a particular embodiment said at least one gene includes: *ZNRF3, TP53, RB1, CDKN2A,* and *CDK4* because of frequent homozygous deletions (*ZNRF3, TP53, RB1*, *CDKN2A*) or amplification (*CDK4*) found for these genes in adrenocortical carcinoma.

In another particular embodiment, in order to better discriminate homozygous deletions from heterozygous deletions, loss of heterozygosity is also searched for chromosome arms 22q, 17p and 9p, carrying *ZNRF3, TP53* and *CDKN2A* respectively.

In another particular embodiment, the analysed 4 CpG-rich regions are Cg07384961, Cg14021073, Cg21494776 and Cg23130254.

As shown in the experimental section using at least 5 SNPs highly heterozygous (heterozygosity close to 0.5 in general population) on each chromosome arm of interest allows to detect gains or losses of just one DNA copy. Then, in a particular embodiment, methods of the invention further comprises analysing at least 5 SNPs per chromosome arm of interest for searching heterozygous deletions or LOH (loss of heterozygosity), said at least 5 SNPs being known to be highly heterozygous in the general population.

In a particular embodiment, the NGS method of the invention when used for the molecular typing of adrenocortical carcinoma, comprises detecting heterozygosity of 11 p and/or of 17p chromosom arms, as it has been shown has having a diagnostic value [49].

In a more particular embodiment, the NGS method of the invention comprises detecting a loss of heterozygosity of 1 p combined with the absence of loss of heterozygosity of 1 q, which is indicative of poor prognosis (data not shown).

As demonstrated in the experimental section and exposed above the method presented herein can be applied to the molecular analysis of many cancers, for which a set of targets is or can be determined by routine methods well known in the art. Furthermore combination in a single targeted NGS experiment and rapid determination of results allow rapid analysis, at high frequency and at lower cost, of patient tumors. It is of particular interest in clinical departments.

Another object of this invention are kits allowing the implementation of the method of molecular typing cancer tumors and, more specifically, of adrenocortical cancer tumors.

In a particular embodiment, invention thus also relates to a kit comprising a single NGS design for analysing (i) specific alterations of chromosome regions, (ii) specific gene mutations, and (iii) DNA methylation status of specific chromosome regions.

The terms "single NGS design" as used herein refers to a single NGS experiment comprising the preparation of two libraries, one for the analysis of the pattern of mutations and for identifying alterations of chromosome regions, and the other for the analysis of DNA methylation status of chromosomes. Advantageously, the two libraries prepared in parallel can be sequenced on the same sequencing chip, following common steps of NGS sequencing. Downstream analysis includes : i) calling mutations in a targeted set of genes, ii) calling chromosome arm alterations through Targomics method especially developed by the inventors (see below), iii) calling DNA methylation status through Targomics. Altogether these targeted measures recapitulate mutations, chromosome status and DNA methylation for each tumor, enabling individual classification into specific molecular classes. As exposed below, such a method is implemented on reduced NGS libraries, wherein only genes or DNA regions that are specific for the molecular typing of the studied tumor are analyzed, and is consequently called hereinafter "Targeted NGS experiment", in contrast to the whole genome or large genomic regions NGS experiments commonly used.

In a more particular embodiment said kit comprises a targeted NGS design comprising one or more primer sets corresponding to at least one gene selected from *HSD3B2, CTNNB1*, *APC, CYP21A2, DAXX, BAI1, CDKN2A, CYP17A1, MEN1, MCAM, RB1, TP53, RNF43, ZNRF3, MED12* and CDK4. In an even more particular embodiment said targeted NGS design comprises one or more primer sets corresponding to at least one gene selected from *ZNRF3, TP53, RB1, CDKN2A,* and *CDK4.* In more specific embodiment said targeted NGS design comprises primer sets corresponding to genes *ZNRF3, TP53, RB1, CDKN2A,* and *CDK4.*

In a particular embodiment said kit comprises a targeted NGS design comprising one primer set corresponding to at least 5 highly heterozygous SNPs located on chromosomes 1 p, 1 q, 2p, 2q, 9p, 11 p, 11 q, 17p, 18p, 18q, and/or 22q.

In a further embodiment the above mentioned kit also comprises a targeted NGS design comprising one or more primer sets corresponding to at least one CpG island selected from Cg07384961, Cg14021073, Cg21494776, Cg23130254, Cg20312228, Cg01635061, Cg27234090, Cg04582938, Cg06039392, Cg10167296, Cg10743104, Cg27425675, Cg16689634, Cg01120165 and Cg15284635. In a very particular embodiment said one or more primer sets comprises at least one CpG island selected from Cg07384961, Cg14021073, Cg21494776 and Cg23130254. In a even more particular embodiment said primer sets corresponds to CpG islands Cg07384961, Cg14021073, Cg21494776 and Cg23130254.

**Brief description of the drawings and tables**

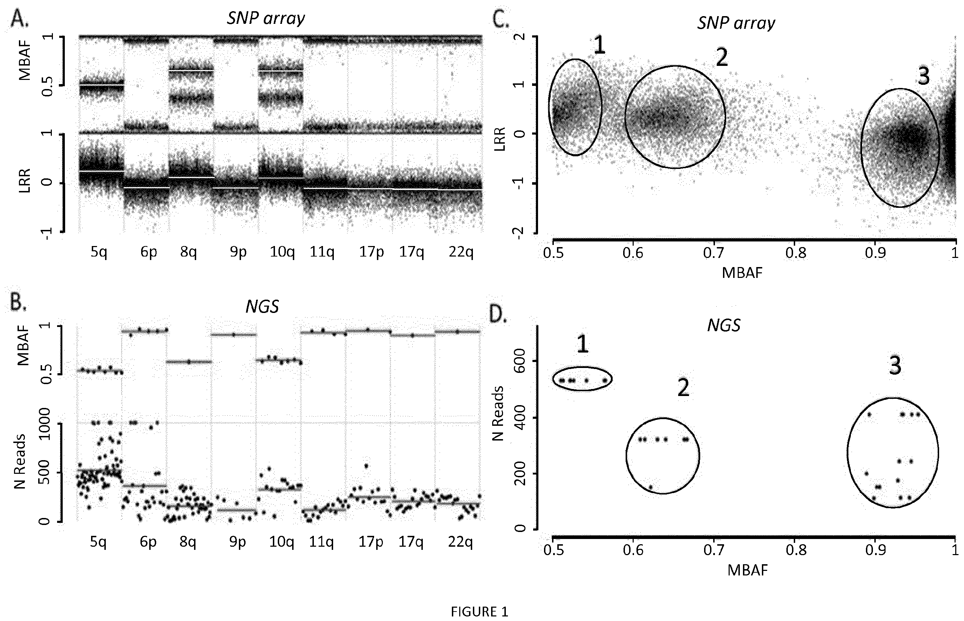

- **Figure 1:** Example of chromosome arms from a tumor with various types of chromosomal alterations: obtaining informations with SNP (panels A and C) and NGS experiments (panels B and D). **A.** With SNP arrays, SNP genotypes are measured by allelic ratios and copy numbers. Allelic ratios (upper panel) are quantified by the BAF (B-allele frequency). BAF is the proportion of alternative - « B »- allele in each SNP genotype. For a normal chromosome arm, heterozygous SNPs (genotype AB) have a BAF of 0.5 -50% of « B » allele in the « AB » genotype. Homozygous SNPs « AA » and « BB » form bands of SNPs with BAFs of 0 -0% of « B » in « AA »-, or 1 -100% of « B » in « BB ». This is illustrated for chromosome 5q, with a band of SNPs centered on BAF=0.5. When chromosomes are lost in almost all tumor cells (6p, 9p, 11 q, 17p, 17q, 22q), the band of heterozygous SNPs is scattered into 2 bands with BAF close to 0 or 1, depending on whether the B or the A allele is lost respectively. When chromosomes are lost in a subset of tumor cells (8q, 10q), these 2 bands show intermediate BAF values, due to the remaining cells with « AB » genotype. MBAF is a simplification of BAF, fitting all BAF values between 0.5 and 1 after « folding » the BAF plot along the 0.5 horizontal axis (formula: MBAF=abs(0.5-BAF) +0.5). MBAF are close to 0.5 for normal chromosomes, close to 1 when chromosomes are lost in almost all tumor cells, and intermediate when chromosomes are lost in a subset of tumor cells. Copy numbers (lower panel) are quantified by LogR ratio. **B.** With targeted NGS, allelic ratios can be quantified by the ratios of read counts of heterozygous SNPs, as examplified on the upper panel. MBAF values are close to MBAF values measured by SNP array. In addition copy numbers can be quantified by read counts of amplicons, as examplified in the lower panel. Read count profiles are close to LRR ratios measured by SNP array. **C.** Scatterplot of allelic ratios (MBAF) and DNA copy number (LRR) generated by SNP array. Three clusters of dots can be identified: cluster 1: a group of SNPs with no allelic imbalance, corresponding to a diploid heterozygous chromosome arm, defining a normal chromosome arm. Thus with this normal chromosome arm, the number of reads corresponding to 2 copies of DNA can be unambiguously deduced; cluster 2: a group of SNPs with some allelic imbalance and a decreased number of reads, corresponding to chromosome arms with loss of one copy in a subclone (~30% of cells); cluster 3: a group of SNPs with almost complete allelic imbalance and a decreased number of reads, corresponding to chromosome arms with loss of one copy in a majority tumor cells. In this latter cluster, MBAF values close to 1 indicate a low tumor contamination by normal cells. **D.** Scatterplot of allelic ratios (MBAF) and DNA copy number (N Reads) generated by NGS. A similar pattern of clusters is observed as in panel C.

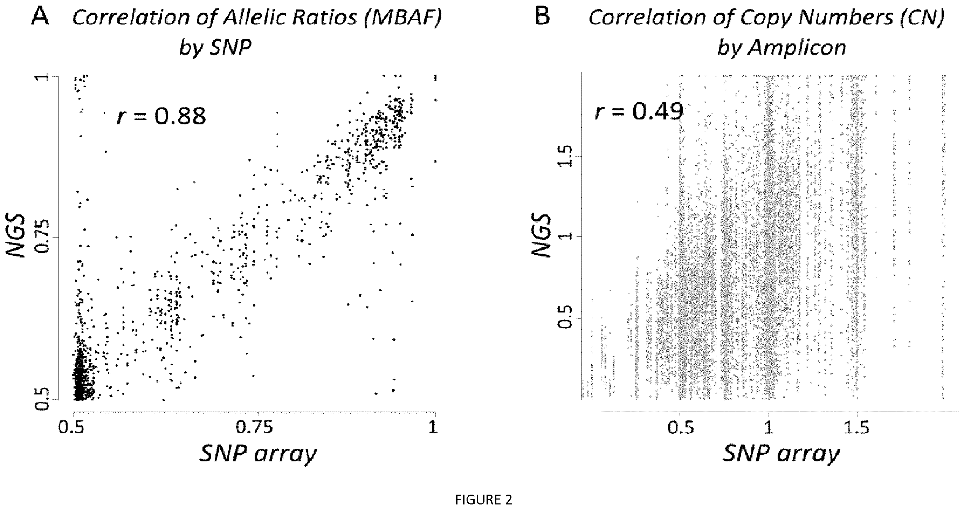

- **Figure 2:** performance of allelic ratios and copy numbers measured by targeted NGS-comparison with SNP arrays. **A.** Correlation of allelic ratios between NGS and SNP arrays. Allelic ratios are quantified MBAFs. **B.** Correlation of copy numbers (CN) between NGS and SNP arrays. CN are expressed relative to ploidy. For instance for diploid cells, CN values of 0.5, 1 and 1.5 correspond to 1, 2 and 3 DNA copies respectively.

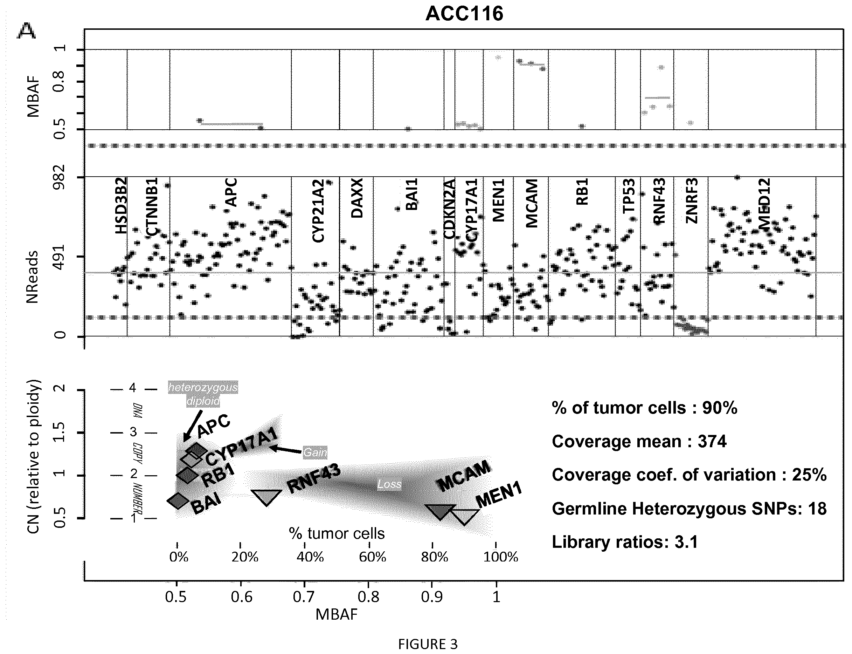

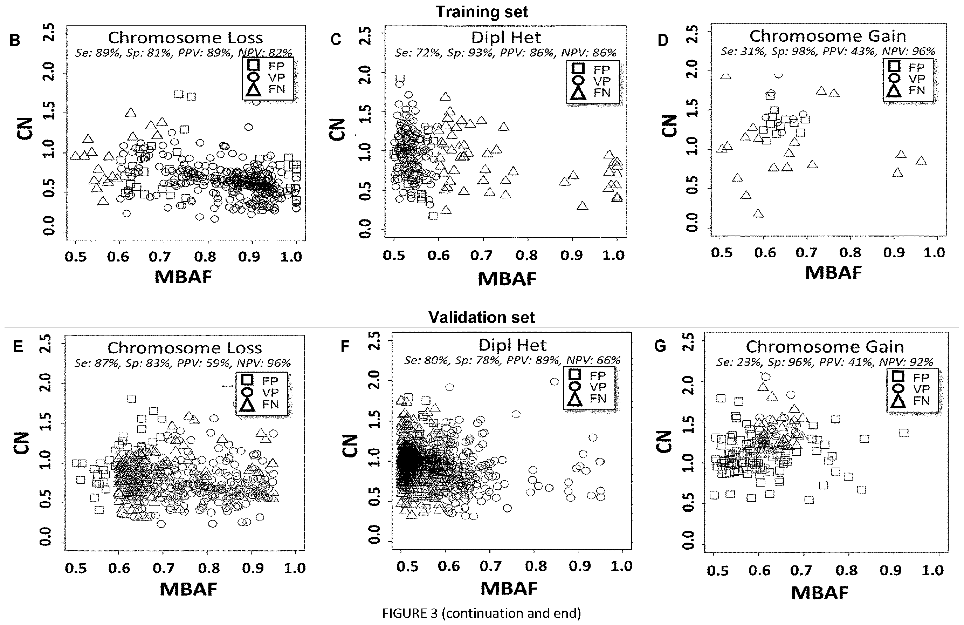

- **Figure 3**: TARGOMICs Automated detection of chromosome alterations from targeted NGS combining copy number (CN) and allelic ratio (MBAF) : **A.** TARGOMICs graphical output from a sample. Upper panel: MBAF of heterozygous SNPs for each gene (line: median value). Intermediate panel: read counts for each gene. Light grey line: mean of read counts. Homozygous deletions are called when read counts drop below a threshold (dashed line; default: 1/3 of mean read of read counts). Lower panel: Scatterplot of genes combining SNP allelic ratios (MBAF, x axis) and amplicon DNA copy number normalized to baseline (CN, y axis). The surface is divided into 3 regions (grey areas), each corresponding to a type of chromosome status (heterozygous diploid, gain or loss). **B, C, D:** performance of TARGOMICs for calling chromosome loss, diploid heterozygous chromosomes, and chromosome gains respectively. Each panel is a scatterplot of genes combining MBAF and CN. False positive, true positive and false negative calls are plotted as squares, circles and triangles respectively. **E,F,G:** performance of TARGOMICs as in B,C,D in the validation set of 449 tumors. *Se: sensibility ; Sp: specifity ; NPV: negative predictive value ; PPV: predictive positive value.*

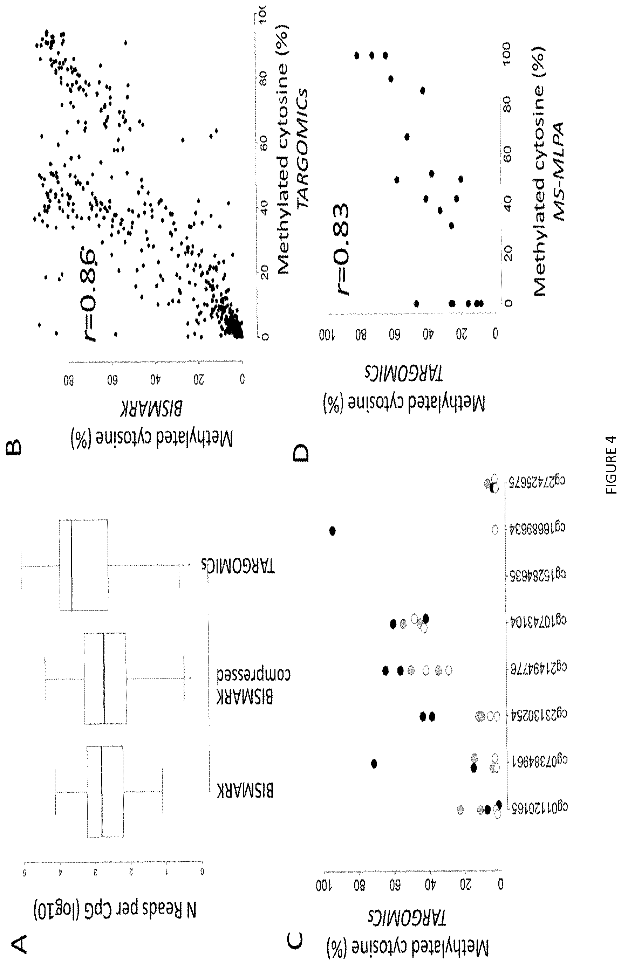

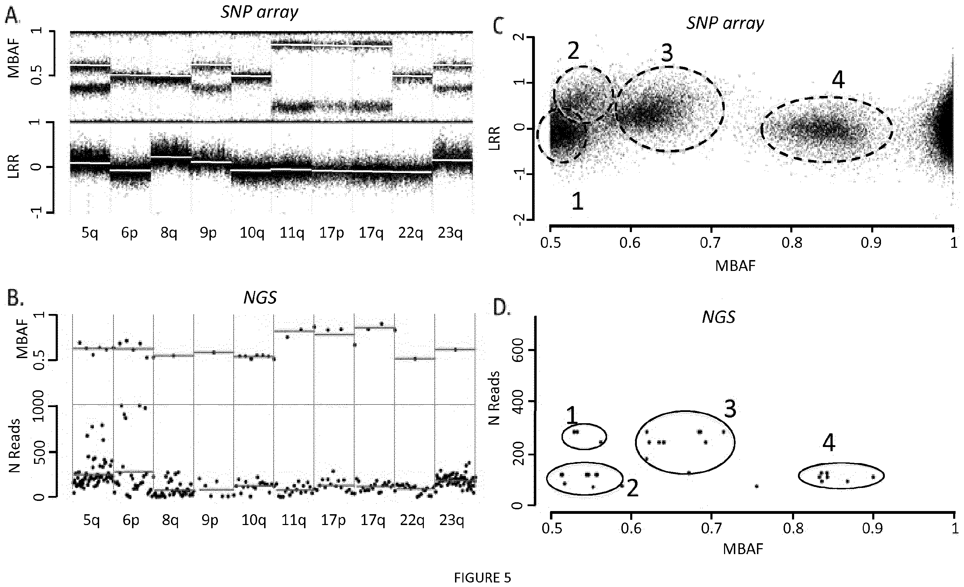

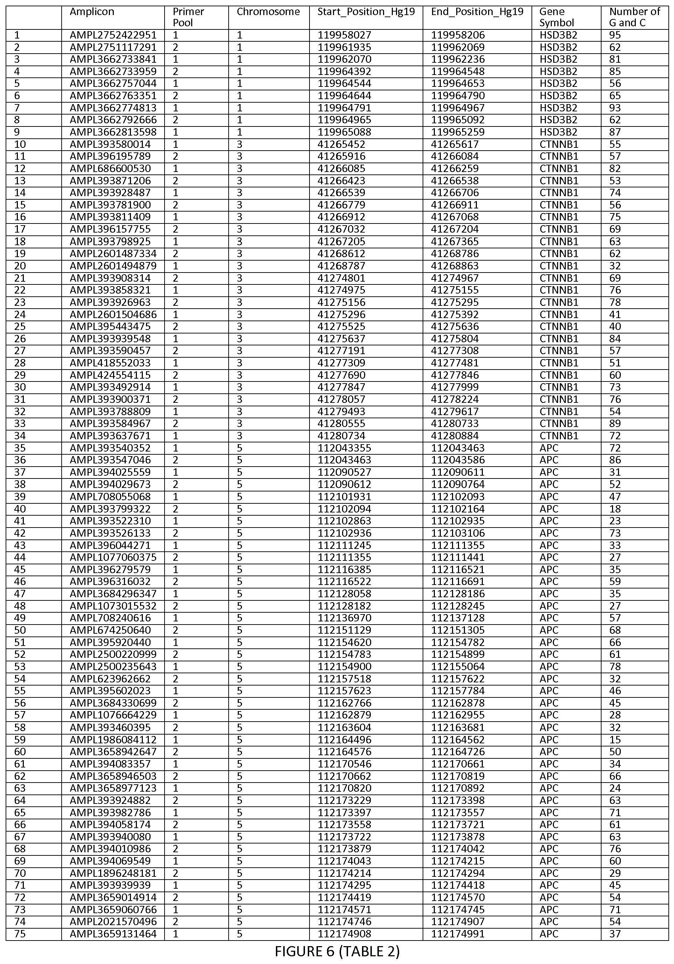

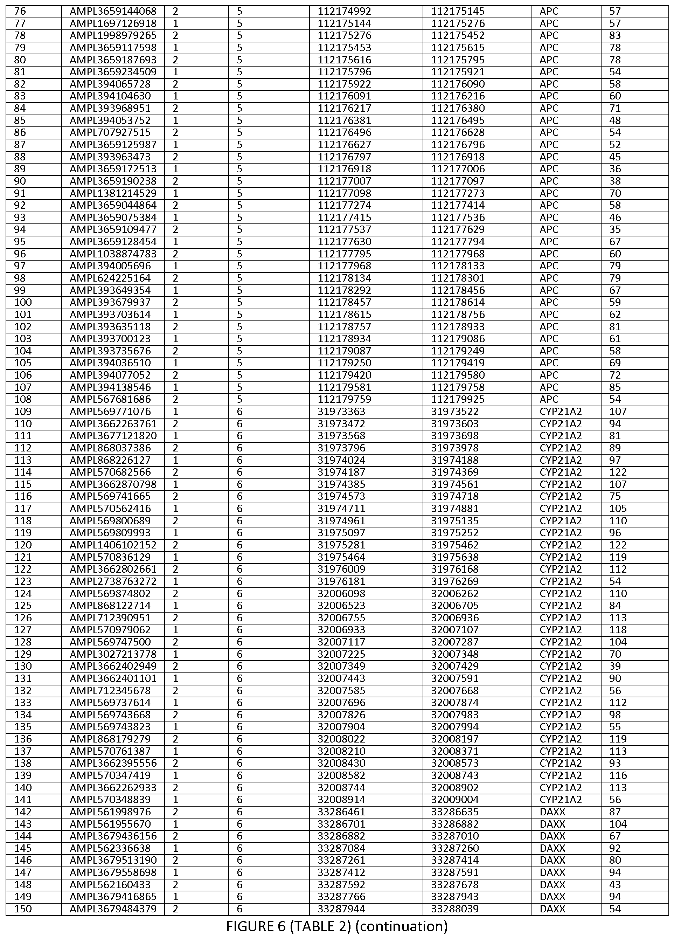

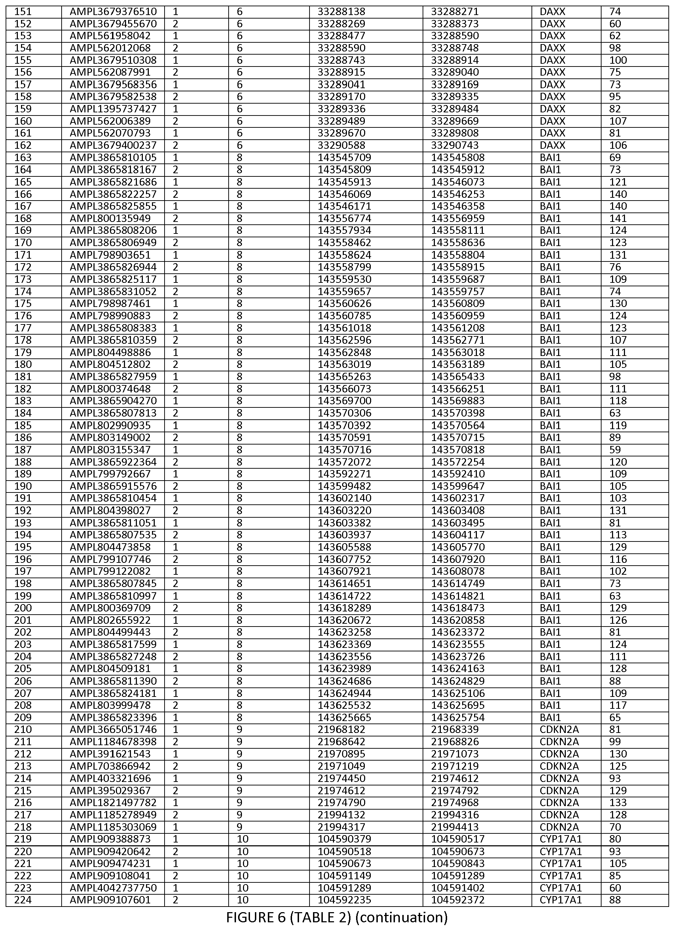

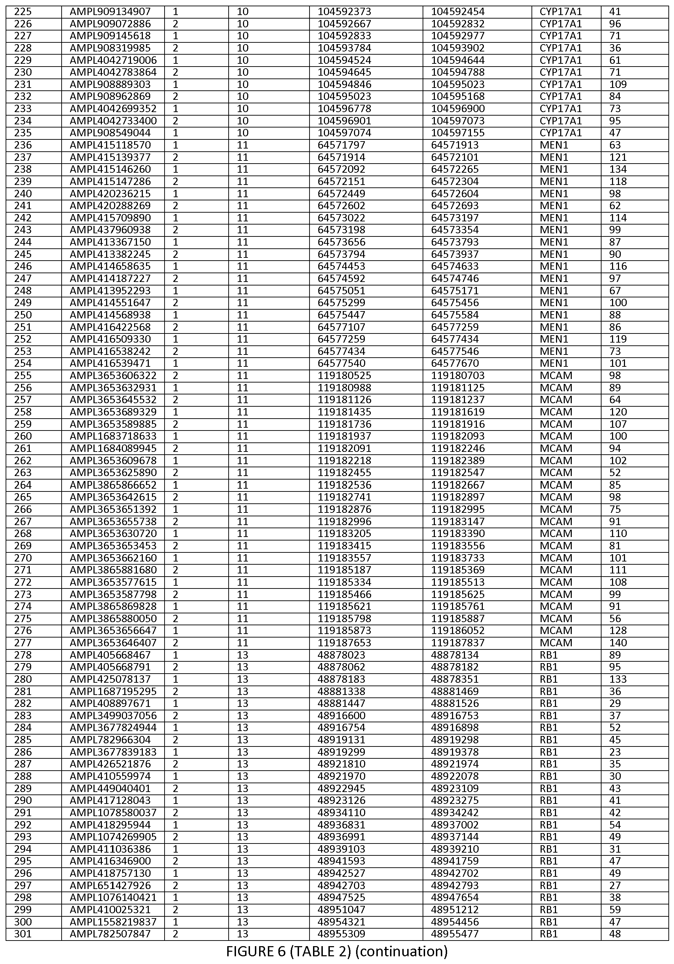

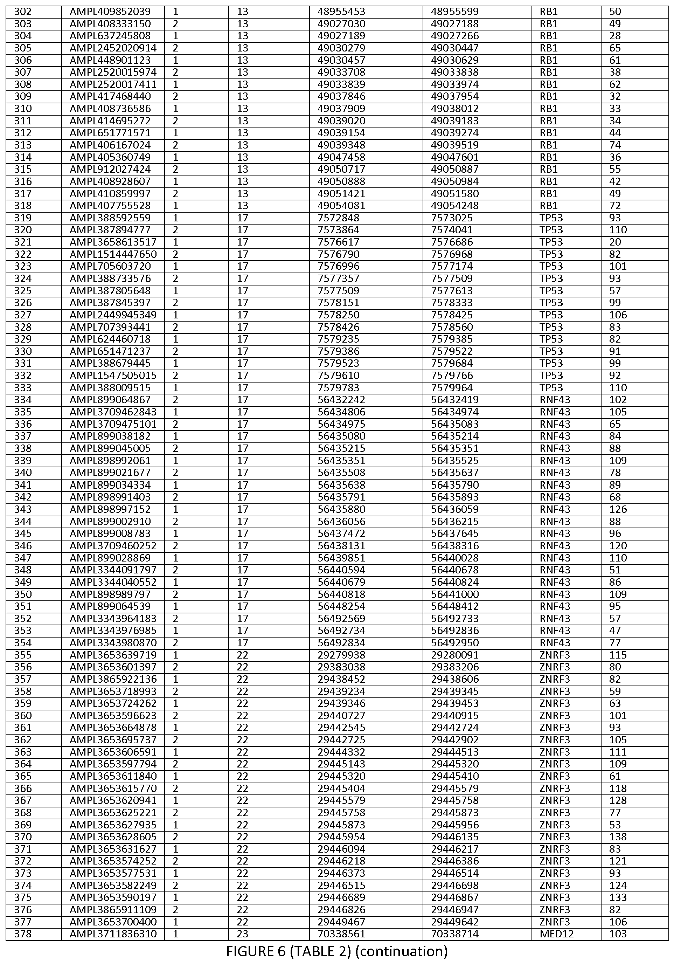

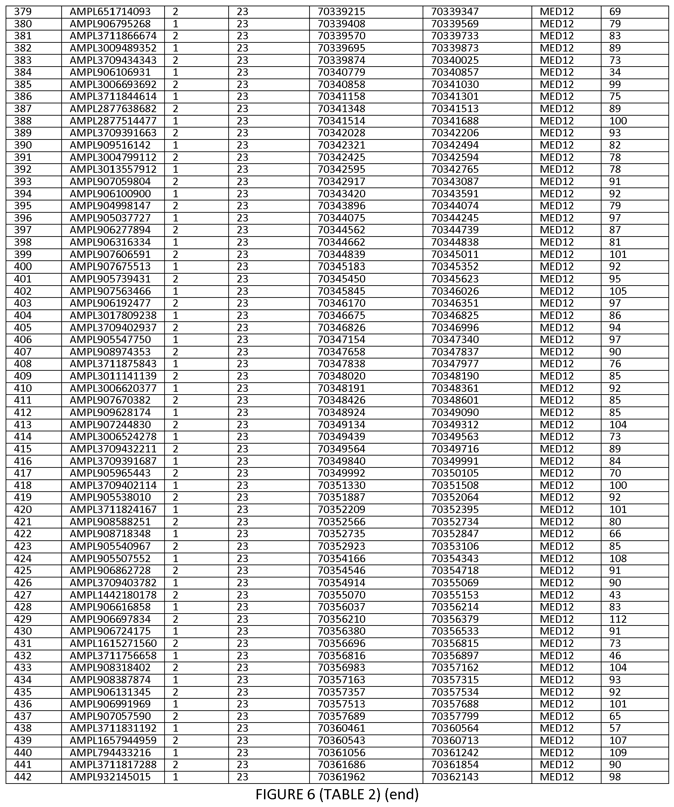

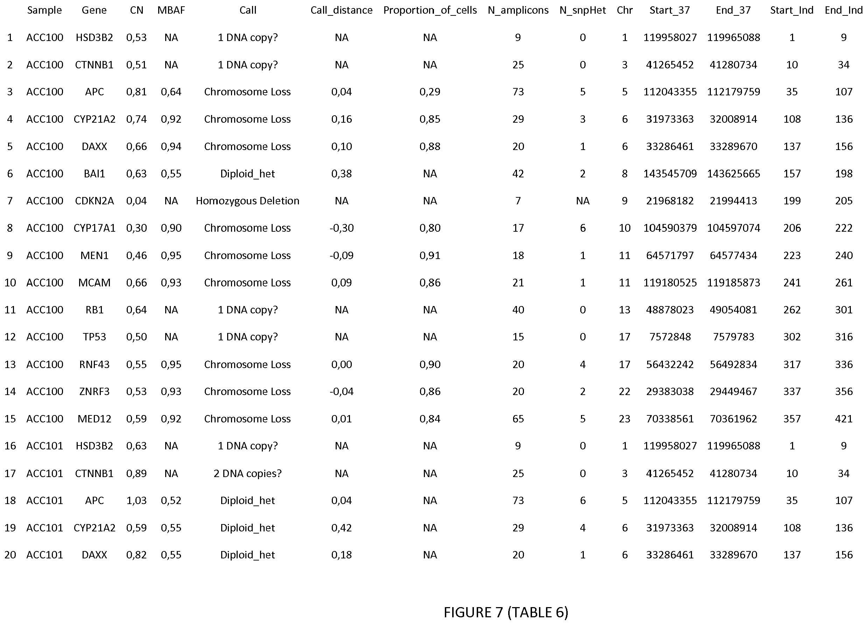

- <u>**Figure 4:**</u> measurement of DNA methylation by targeted NGS: **A.** Comparison of CpG coverage using BISMARK (diffuse seeding alignment), BISMARK after compressing the stretches of homopolymers, and with TARGOMICs (seeding alignment restricted to the primers 3' end, compression of homopolymers). **B.** Correlation between the proportion of methylated CpGs generated by BISMARK and TARGOMICs. **C.** Proportion of methylated CpGs in 6 tumors characterized by methylation array. Methylation array could classify tumors into high methylation (CIMP-high; black dots), intermediate methylation (CIMP-intermediate; grey dots) or not hypermethylated (non CIMP; white dots). CIMP high and CIMP intermediate showed higher proportions of methylation in most of CpG islands (x axis). **D.** Proportion of methylated CpGs in 26 tumors, measured either by TARGOMICs (y axis) or by MS-MLPA (x axis) are strongly correlated.
- <u>**Figure 5**</u>: combining allelic ratios and DNA copy number allows to detect chromosome arms alterations and tetraploidy in a tumor. **A.** SNP array profiles of allelic ratios (MBAF) and copy numbers (LRR) show various levels of alterations. **B.** With targeted NGS, SNP *allelic ratios* (MBAF) deduced from *ratios of read counts* of heterozygous SNPs, and amplicon copy numbers deduced from *read counts* show similar patterns compared to SNP array. **C.** Scatterplot of allelic ratios (MBAF) and DNA copy number (LRR) generated by SNP array. Four clusters of dots can be identified: clusters 1 and 2: two groups of SNPs with no allelic imbalance (MBAF close to 0.5), but distinct copy numbers, corresponding to a diploid heterozogous chromosome arm (cluster 1 ; genotype « AB ») and a tetraploid chromosome arm (cluster2 ; genotype « AABB »); cluster 3: a group of SNPs with some allelic imbalance and a number of reads between cluster 1 and cluster 2, corresponding to heterozygous chromosome arms with gain of one copy (3 copies of DNA ; genotypes « AAB » and « ABB »); cluster 4: a group of SNPs with almost complete allelic imbalance and a number of reads corresponding to cluster 2, corresponding to homozygous chromosome arms with 2 copies of DNA (2 copies of the same allele ; genotypes « AA » or « BB »). In this latter cluster, MBAF values close to 0,85 indicate some tumor contamination by normal cells (around 30% of cells). **D.** Scatterplot of allelic ratios (MBAF) and DNA copy number (N Reads) for heterozygous SNPs, generated by NGS. A similar pattern of clusters is observed as in panel C.

- Table 2 represents NGS panel design.
- Table 6 represents Example of chromosome status called by TARGOMICs.

« Calls » generated automatically by Targomics include « diploid heterozygous » (for normal chromosomes), « chromosome loss » or « chromosome gain » when heterozygous SNPs are available; « 0- », « 1- », « 2-», « 3-», or « 4- DNA copies » when no heterozygous SNP is available. DNA copy number is then inferred only from read counts and thus is less reliable; « homozygous deletion » and « high level amplification » for major shifts of DNA copy numbers.

Other columns : « CN »: copy number determined from amplicons read counts, expressed relative to baseline (1: 2 copies, 0.5: Chromosome loss, 1.5: chromosome gain) ; MBAF: median of MBAFs for all heterozygous SNPs of one gene ; « Call_distance »: euclidian distance to the center of each region corresponding to chromosome alterations in the MBAF/CN scatter plot (see figure 3A) ; « Proportion_of_cells »: proportion of cells with the chromosome alteration (deduced from the MBAF) ; « N_amplicons »: number of amplicons in the gene ; « N_snpHet »: number of heterozygous SNPs in the gene ; « Chr », « Start_37 », « End_37 »: physical positions ; « Start_Ind », « End_Ind »: index position in the targeted NGS data set.

## EXAMPLES

### EXAMPLE 1: MATERIAL AND METHODS

#### A- <u>Samples</u>

A training set of 109 adrenocortical carcinoma samples was analyzed, including 77 both sequenced by targeted NGS (tumor and leucocyte) and analyzed by SNP arrays (tumors), and 32 sequenced by NGS after bisulfite treatment, 6/32 were also analyzed by methylation array, and 20/32 were also analyzed by MS-MLPA (see below).

These tumors were snap frozen early after surgery, and kept in liquid nitrogen until use. DNA extraction was performed using standard protocols as previously described [37].

A validation set of 449 cancer samples, from 42 distinct cancer types was analyzed (Table 1), both sequenced by targeted NGS and analyzed by SNP array [50].

**B- SNP and methylation arrays**

In the training set, SNP array and methylation array experiments were performed using the Illumina HumanCore-12v1 and the Illumina Human Meth27 Beadchips respectively, following the manufacturer recommandations as previously described [38].

In the validation set, 449 additional SNP arrays (Affymetrix Cytoscan HD, Santa Clara) were also included as a validation cohort, generated in the SHIVA study [50].

Chromosome alterations were called using GAP [39]. Chromosome alteratoins were validated graphically, visualizing logR ratio (LRR) and B-allele frequency (BAF) SNPs along the genome (see figure 1A for example). Briefly, calling A and B the 2 alleles, BAF is calculated to reflect the genotype: BAF is 0 for genotypes *AA,* 0.5 for genotypes *AB,* and 1 for genotypes *BB.* BAF can be summarized by the following formula:

$$BAF = Signal_B / (Signal_A + Signal_B)$$

For Illumina SNP arrays, BAF is directly provided. Segments were averaged into a single call for each chromosome arm. For Affymetrix SNP arrays, BAF was calculated for each segment called by GAP [39], combining allelic difference Y and copy number CN in the following formula:

$$BAF= (Y+CN)/(2xCN).$$

This formula is deduced from the definition of Y and CN. Indeed, Y is the substraction of signals from allele A and allele B:

$$Y= log(B)-log(A).$$

And the copy number is the addition of signals from allele A and allele B:

$$CN = log(A) + log(B)$$

CN was adjusted to be centered on 1 instead of 0 (1: 2 copies of DNA, 0.5: 1 copy, 1.5: 3 copies of DNA).

**C- Targeted NGS sequencing**

1- Generating *read counts* and SNP genotypes (training set)

In the training set of 77 adrenocortical carcinoma, multiplex PCR was performed targeting a panel of 15 genes, sequenced following the manufacturer recommendations (Table 2). Briefly, these 15 genes were amplified with 442 primer pairs -covering 66,266 bp- from two pools of multiplex primers designed with the AmpliSeq Designer V2.0 (Thermofisher, Villebon sur Yvette, FRA). Libraries were massively-parallel sequenced by semiconductor sequencing technology on a PGM (ThermoFisher).

2- Generating *read counts* and SNP genotypes (validation set)

In the validation set of 449 cancers, commercial cancer panels designed for the screening of hotspot mutations were used, either the Ion Ampliseq cancer panel V1, or the Ion Ampliseq cancer panel V2 (ThermoFisher). Libraries were generated with the Ampliseq library kit v2·0 and sequenced on a PGM (ThermoFisher) [50].

3- Sequencing PCR products of bisulfite-treated DNA

For all samples, 2µg of Tumor DNA were used for the bisulfite treatment by EZ DNA Methylation -Gold Kit (Zymo Research,CA, USA) following the manufacturer protocol. Bisulfite treatment transforms unmethylated cytosines in thymines. The bisulfite treated DNA was then amplified by PCR using methyl-insensitive primers, designed by Methprimer [40]. The list of probes is provided in Table 3 below. Bisulfite-treated DNA was amplified by PCR with the TaqGold (ThermoFisher), with the following program: 8 min at 95°C, 40 cycles of 1 min at 95°C, 1 min at 58°C, 1 min at 72°C and a final extension of 8 min at 72°C. For each tumor, the different PCR products -corresponding to different CpG islands-were mixed together. A single NGS library was then generated with the Ion Plus Fragment Library Kit (ThermoFisher) following the manufacturer recommendations, except for the replacement of end-repair procedure -the End-repair enzyme-(Thermofisher) was replaced by End-It™ DNA End-Repair Kit (Epicentre, Madison).

Table 3

| CpG island | Chromosom | Start Position (Hg19) | End Position (Hg19) | Surrounding genes (<10kbp) | Forward Primer | Reverse Primer | Number of CpG |
|---|---|---|---|---|---|---|---|
| cg20312228 | chr3 | 126113521 | 126113740 | CCDC37 | GGAGTGTTGAATTTTAGAGGAAGAAATT (SEQ ID NO : 1) | CCAAAACAACCATTCTTCAAACTAACTATA (SEQ ID NO : 2) | 17 |
| cg01635061 | chr6 | 33160821 | 33160933 | COL11A2 | TAGGTTTAAGGAGATGTAAATGGGGGA (SEQ ID NO : 3) | CCCTAAACCCTTCCAACCAAAATC (SEQ ID NO : 4) | 5 |
| cg27234090 | chr11 | 119252336 | 119252587 | USP2-AS1 | GGGGGTTTAGAAGGGATTTTTT (SEQ ID NO : 5) | CCACCTCTATTCCTACTCCACCC (SEQ ID NO : 6) | 23 |
| cg04582938 | chr9 | 139971592 | 139971711 | UAP1L1 | GGGGTTTGGAGAAGAGTAGGAA (SEQ ID NO : 7) | CCCAACCCCATCTCTCCAACCTATC (SEQ ID NO : 8) | 7 |
| cg06039392 | chr4 | 187476256 | 187476395 | MTNR1A | AAGTGTTTGGGGAAGGTTGGTTGTT (SEQ ID NO : 9) | CTAAACAACCTCCTAATCATCCTATCC (SEQ ID NO : 10) | 12 |
| cg14021073 | chr11 | 1410094 | 1410283 | BRSK2 | TTTAGTTGTTTAATTTGAAAGAGGGGT (SEQ ID NO : 11) | AAAAATTATACACCCCCAAAAAAAAC (SEQ ID NO : 12) | 16 |
| cg10167296 | chr1 | 206680385 | 206680580 | RASSF5 | GGAGAGAGGAAGGGTTAAGGAGTA (SEQ ID NO : 13) | CAATCACTTTCCCCAACACCAAATTC (SEQ ID NO : 14) | 14 |
| cg10743104 | chr1 | 13909575 | 13909779 | PDPN | GTGAATTAGGTTTGGAAGGGGATATA (SEQ ID NO : 15) | CCCCAAAACTAACTAATAAAAAAATTTAACA (SEQ ID NO : 16) | 13 |
| cg23130254 | chr2 | 176964015 | 176964228 | HOXD12 | TTGGGGTAAAAGTGATATTGTTTAGGT (SEQ ID NO : 17) | CTACCCAAATATCCCCTAAAACTCTTC (SEQ ID NO : 18) | 15 |
| cg27425675 | chr16 | 11036627 | 11036797 | DEXI | TTTGTTGTATGTTTTTTTGGGATTT (SEQ ID NO : 19) | AACCTTCCAACAACCAAAATTTAAA (SEQ ID NO : 20) | 8 |
| cg16689634 | chr1 | 47489490 | 47489660 | CYP4X1 | TATGGAATTTTTTTGGTTGGAGA (SEQ ID NO : 21) | TACCCAAAAAACCAATAAATAAAAAAC (SEQ ID NO : 22) | 11 |

(continued)

| CpG island | Chromosom | Start Position (Hg19) | End Position (Hg19) | Surrounding genes (<10kbp) | Forward Primer | Reverse Primer | Number of CpG |
|---|---|---|---|---|---|---|---|
| cg01120165 | chr22 | 50689136 | 50689364 | HDAC10 | GTTTTTAGGTTTTTAGTTGGGTTGTT (SEQ ID NO : 23) | CTCTAAACTAAACTCCTCATCTACCCC (SEQ ID NO : 24) | 18 |
| cg15284635 | chr3 | 10857661 | 10857863 | SLC6A11 | TTTGGTTTAGTAGGTTAGTGGGTAGG (SEQ ID NO : 25) | AAAAAAACAAAAAATAAAACTAAAAAACC (SEQ ID NO : 26) | 22 |
| cg21494776 | chr19 | 10398549 | 10398731 | ICAM4 | GAAGGGGGTAGAGAGAGTTATGATTT (SEQ ID NO : 27) | CAATAAAAAAAATCCCTACAAAAACAAC (SEQ ID NO : 28) | 12 |
| cg07384961 | chr16 | 3068252 | 3068465 | CLDN6 | GAGGGGTAGAGATTTTGTTTTTGA (SEQ ID NO : 29) | AAAATTAAATAAATTCCCCATATCACC (SEQ ID NO : 30) | 13 |

**D- Treatment of NGS data**

A set of original scripts optimized for targeted NGS data was specifically developed, gathered under the name "TARGOMICs", which makes use of read count, allelic ratio, allelic ratio for heterozygous SNPs, as well as identified methylated regions.

1- Getting read count, allelic ratio, and allelic ratio for heterozygous SNPs.

*Getting read count*

For each amplicon, the number of reads properly aligned were extracted using IonTorrent suite v3.6.2, using the Coverage Analysis plugin (Thermofisher).

Amplicons and samples with a mean coverage <30 reads per amplicon were discarded. For the remaining amplicons, for each sample, the 2 libraries were normalized to reach an equal mean number of reads by amplicon (see below).

*Getting allelic ratios*

Allelic ratios are expressed as MBAF.

For each heterozygous SNP, calling A and B the 2 alleles, the proportion of B-allele, also called "BAF" (B-allele frequency), can be calculated to reflect the genotype: BAF is 0 for genotypes *AA,* 0.5 for genotypes *AB,* and 1 for genotypes *BB.* BAF was determined from the read counts ($N_{reads}$), using the following formula:

$$BAF_{SNP} = N_{reads\ allele\ B} / (N_{reads\ allele\ A} + N_{reads\ allele\ B})$$

*Allelic ratios are then normalized as Mirror B Allele Frequencies (MBAF[39]):* BAFs -which span between 0 and 1-, were then converted into MBAF -which span from 0.5 to 1-, by applying this formula:

$$MBAF_{SNP} = Abs(BAF_{snp} - 0.5) + 0,5$$

*Selecting heterozygous SNPs*

In the training set, leucocytes were sequenced for each patient, in addition to tumors. Therefore, heterozygous SNPs were identified from the leucocyte genotypes, considering SNPs with MBAF<0.6. These SNPs were subsequently studied in the tumor, computing the MBAF from the reads in the tumor.

In the validation set, no leucocyte data were available. To exclude germline homozygous SNPs, all SNPs with an MBAF≥0.95 were excluded. Among the remaining SNPs, the next step was to discriminate germline heterozygous SNPs - the informative SNPs-, from somatic mutations -which are particularly numerous in cancers when using NGS panels optimized for catching somatic mutations hotspots. For that aim, only the SNPs commonly found in general population, using a >5% threshold, were considered.

2- Calling "normal", "gained" and "lost" chromosomes (TARGOMICs)

Each gene was defined as an independent chromosome segment.

*Allelic ratio* for each gene was obtained by averaging the MBAF of heterozygous SNPs of each gene.

*Read counts* for each gene were converted into relative copy number (CN), i.e. relative to a baseline CN. This baseline was determined for each sample as a set of "normal genes", *i.e.* with 2 copies of DNA and no allelic imbalance (the above heterozygote SNPs). The first step was to identify the CN shared by a maximum number of genes. For that aim, the number of reads of amplicons were compared for each gene, using a Student t-test. Genes with no significant differences (p>0.05) were considered as identical in terms of CN. The maximum number of genes sharing an identical CN were identified as "baseline genes". A baseline read count was calculated as the mean read counts of "baseline genes". All read counts were subsequently divided by the baseline read count, thus generating relative CN -i.e. relative to baseline-. The second step was to check whether the "baseline genes" also showed no allelic imbalance -i.e. showed an MBAF close to 0.5 (<0.6)-. If no baseline gene verified this condition, genes with higher or lower relative CN and MBAF close to 0.5 were sought. If found, the baseline CN was shifted to these genes: all relative CN were then divided by the relative CN of these genes.

For each sample, a scatterplot of all genes was subsequently generated, with MBAF in Y-axis and relative CN in X-axis. The scatterplot was divided into distinct regions, corresponding to each type of chromosome status: "normal" chromosomes for MBAF close to 0.5 with relative CN close to 1; "lost" chromosome for MBAF >0.5 with relative CN <1; "gained" chromosome for MBAF >0.5 and <0.67 with relative CN>1 (figure 3A).

3- Calling homozygous deletions and high level amplifications (TARGOMICs)

Homozygous deletions were called for any gene with a relative CN lower than a threshold (default value: 0.3). For detecting subregions of genes with homozygous deletions, the following algorithm was applied: any region with *n* (default value: 9) or more consecutive amplicons reaching a relative CN lower than TARGOMICs' threshold (default value: 0.3) were identified as deleted segments.

High CN amplifications were called for any gene with a mean relative CN higher than TARGOMICs' threshold (default value: 3).

4- Aligning bisulfite-treated DNA and counting the methylated cytosines

*Alignment and counting with TARGOMICs*

A specific alignment script was originally created. For each alignment, two specific reference sequences are generated by *in silico* bisulfite treatment, one for the methylated allele -cytosines of CpGs remain cytosines-, and one for the unmethylated allele -cytosines of CpGs are replaced by thymines.

For each CpG island sequenced, several steps are performed:

1. Compression of all homopolymers into a single base, except around the CpGs - the dinucleotides CG, TG and CA are systematically excluded from this compression-. This enables to get rid of the numerous indel artefacts generated by semiconductor sequencing occurring within homopolymers and decreasing the mismatching of the sequences during the alignment.
2. Identification of reads aligned on the 3' end of each primer (by default the 15 last bases), using the forward and complementary reverse sequences. This allows for the selection of reads, their proper alignment and orientation.
3. Testing each read aligned on primers for its alignment on reference sequence, using the methylated reference -excluding the CpG positions-, tolerating a maximal error rate (10% by default).
4. Counting C and T alleles for each CpG from all properly aligned reads. The proportion of C reflects the proportion of methylated cytosins for each CpG (not shown).

*Alignment and counting with* BISMARK

For the purpose of comparison, Aligning bisulfite-treated DNA alignment and counting of the methylated cytosins were performed using BISMARK [41]. Two methods were applied, based on the use of Bismark (Bismark v0.16.1; dependancy: Bowtie2 version 2.2.9; default settings): (i) with CpG islands sequences after *in silico* transformation of cytosines in thymines reflecting bisulfite DNA treatment; (ii) with similar sequences, but after compression of homopolymers (see above). Information was extracted CpG by CpG with bismark_methylation_extractor (parameters: "--scaffold --split_by_chromosome --comprehensive --bedGraph - counts").

*Optimization of CpGs selection*

The most informative CpGs were selected. For that aim, a training set of 6 tumors was used, studied both by methylation array and by NGS. Only the CpGs with a significant and positive correlation between NGS measure and the global methylation measured by methylation array (global methylation is defined as the mean M-Value calculated for each tumor from the top 1000 probes with the highest standard deviation among adrenocortical carcinomas [51], not shown) were considered. These CpGs were normalized and averaged for each CpG island. These CpG are Cg07384961, Cg10743104, Cg21494776, Cg23130254

*Methylation specific-multiplex ligation-dependent probe amplification (MS-MLPA) experiments*

20 adrenocortical carcinoma were analyzed by MS-MLPA to validate the proportions of methylated CpGs as determined by TARGOMICs, using the SALSA MLPA ME002 tumor suppressor-2 probe mix, combined with the SALSA MLPA EK1-Cy5 or EK1-FAM reagent kits (MRC-Holland). Methylation level was deduced for each sample from the average methylation level of 4 genes (GSTP1, PYCARD, PAX6, PAX5), as previously described [51].

5- Bioinformatic Codes

All the scripts were programmed in R (www.r-project.org). TARGOMICs source codes are freely available for research use only but codes have been filed for commercial uses.

## EXAMPLE 2: RESULTS

### Informations obtained from NGS *allelic ratios* and *read counts* regarding somatic chromosomal alterations

A chromosome loss can be detected either by a decreased DNA copy number (CN), or by loss of heterozygosity (LOH; Figure 1). LOH is an extremum of allelic imbalance (AI), where one allele is completely lost [42] (Figure 1). Using NGS, *read counts* for each amplicon should somehow reflect DNA CN. Similarly, for heterozygous SNPs, ratios of read counts measured for each allele, termed *allelic ratios,* should reflect AI. This was tested in a training set of 77 adrenocortical carcinoma tumors genotyped both by SNP array and NGS, using SNP array data as a gold standard (Figure 1).

Indeed, using NGS and considering all heterozygous SNPs, *allelic ratios* globally were found as strongly correlated with AI measured by SNP array (Pearson r=0.88, $p<10^{-12}$, Figure 2A). Similarly, *read counts* of NGS amplicons also correlated with CN determined by SNP array. However compared with *allelic ratios,* correlation coefficient was weaker (Pearson r=0.49; $p<10^{-12}$, Figure 2B).

Based on these correlations, between NGS and the gold standard (SNP array), simulations have been performed to test the ability of NGS *allelic ratios* and *read counts* to discriminate AI and CN respectively. Concerning *allelic ratios* (normalized as MBAFs, which range from 0.5 for unaltered heterozygous SNPs to 1 for SNPs with complete LOH (Figure 1A and 1B)), variations of 0.2 were detectable with one single SNP, and variations of or below 0.1 were detectable with at least 4 consecutive SNPs (Table 4).

Table 4

| | | **Detectable MBAF variation** | | | | | | | | | |
|---|---|---|---|---|---|---|---|---|---|---|---|
| **Number of NGS SNP** | | **0.04** | **0.06** | **0.08** | **0.1** | **0.12** | **0.14** | **0.18** | **0.2** | **0.25** | **0.3** |
| | 1 | 6314 | 6962 | 7567 | 8025 | 8532 | 8829 | 9387 | 9563 | 9827 | 9944 |
| | 2 | 6854 | 7678 | 8311 | 8887 | 9261 | 9555 | 9846 | 9925 | 9985 | 10000 |
| | 3 | 7271 | 8169 | 8894 | 9311 | 9623 | 9789 | 9975 | 9987 | 10000 | 10000 |
| | 4 | 7492 | 8495 | 9134 | 9579 | 9808 | 9920 | 9994 | 9997 | 10000 | 10000 |
| | 5 | 7833 | 8719 | 9372 | 9749 | 9900 | 9963 | 9998 | 9999 | 10000 | 10000 |
| | 6 | 8055 | 8958 | 9524 | 9844 | 9946 | 9991 | 9997 | 10000 | 10000 | 10000 |
| | 7 | 8201 | 9105 | 9633 | 9890 | 9973 | 9997 | 9999 | 10000 | 10000 | 10000 |

*In grey : MBAF variations are properly detected in >95% of cases*

Concerning *read counts* (normalized as relative CNs, with "1" for 2 DNA copies, and "0.5" for chromosome losses (1 DNA copy) in a pure tissue of diploid cell) variations of 0.5 were detectable with five amplicons, and variations of 0.25 with at least 20 amplicons (Table 5).

Table 5

| | | Detectable CN variation | | | | | | | | | |
|---|---|---|---|---|---|---|---|---|---|---|---|
| Number of NGS | | | **0.1** | **0.12** | **0.14** | **0.18** | **0.2** | **0.25** | **0.3** | **0.4** | **0.5** |
| | | **1** | 5709 | 5683 | 5750 | 6062 | 6167 | 6505 | 6775 | 7257 | 7655 |
| | | **5** | 6240 | 6582 | 6757 | 7299 | 7504 | 7972 | 8386 | 9055 | 9531 |
| | | **10** | 6809 | 7060 | 7513 | 8061 | 8336 | 8831 | 9245 | 9703 | 9895 |
| | | **15** | 7208 | 7583 | 7940 | 8531 | 8732 | 9197 | 9573 | 9881 | 9984 |
| | | **20** | 7431 | 7916 | 8210 | 8819 | 9084 | 9515 | 9776 | 9952 | 9991 |
| | | **30** | 7990 | 8308 | 8739 | 9272 | 9502 | 9795 | 9925 | 9994 | 9999 |
| | | **40** | 8291 | 8637 | 9092 | 9572 | 9688 | 9899 | 9976 | 9998 | 10000 |
| | | **50** | 8501 | 8954 | 9301 | 9707 | 9827 | 9951 | 9993 | 10000 | 10000 |
| | | **75** | 8959 | 9365 | 9649 | 9886 | 9944 | 9996 | 10000 | 10000 | 10000 |
| | | **100** | 9306 | 9594 | 9811 | 9961 | 9976 | 10000 | 10000 | 10000 | 10000 |

*In grey : CN variations are properly detected in >95% of cases.*

It was found that *read counts* may be efficient for detecting homozygous deletions (loss of the two DNA copies), and high level amplifications (data not shown). However *read counts* did not perform well for detecting chromosome losses or gains of one copy, especially in heterogeneous tissues -such as tumor samples. It was also found that *allelic ratios* were more robust than *read counts,* and should therefore be used as main information for calling chromosome gains or losses of just one DNA copy. Indeed, *allelic ratios* detect properly losses of one DNA copy. More specifically, MBAF (calculated from *allelic ratios,* see above) increases from 0.5 to 1 for one DNA copy loss is occurring in all cells. In heterogeneous tissues, MBAF increase is lower but remains important, for instance from 0.5 to 0.75 in case of losses occurring in half of cells. Of note, detecting chromosome gains was not as efficient as detecting chromosome losses, despite the use of MBAF. This is related to a more limited impact of chromosome gains on *allelic ratios.* Indeed a chromosome gain from two to three DNA copies is associated with a MBAF increase from 0.5 to 0.666 when the gain occurs in all cells. As soon as contaminating cells are present, this shift drops to lower values, barely detectable. For instance, for a chromosomal gain in half of the cells, MBAF shifts from 0.5 to 0.583.

Calling chromosome gains or losses of just one DNA copy implies that heterozygous SNPs are available for each chromosome of interest. Based on original simulations (Table 4), the method includes several SNPs as internal control (at least 5 to 10 per chromosome arm) with high heterozygosity in population (close to 0.5) for each chromosome arm of interest, when designing targeted NGS panels. Such a low number of SNPs will not dramatically increase the cost of the design nor of the sequencing, and will be very effective for reliable calls of chromosome alterations and particularly when talking about detecting loss of one DNA copy. In this study heterozygous SNPs were identified from sequencing patients' leucocytes -in addition to tumors-, using the same NGS panel. The advantage is to catch all heterozygous SNPs, including those with low heterozygosity in population, and independently of their *allelic ratio* in tumor. Alternatively, it is possible to choose 5 to 10 SNP per chromosome arm, instead of realizing leucocyte sequencing, to avoiding this cost spending step. The present analysis demonstrated that *allelic ratios* are precise -MBAF variations of 0.1 are detectable-. However some artefact calls can reach high *allelic ratios.* These artefacts may be filtered out by including a few (5 to 10) SNPs for each chromosome arm, with high heterozygosity in population. Indeed such a combination of SNPs can precisely estimate chromosome arm *allelic ratio,* and therefore provide an expected *allelic ratio* for all heterozygous SNPs on this chromosome arm.

Allelic ratios are thus more informative than read counts for detecting somatic chromosomal alterations.

To confirm these findings, correlations between NGS and SNP array were further tested in an independent cohort of 449 tumors from 42 distinct tumor types (Table 1). Allelic ratios measured by NGS were found to strongly correlate with AI measured by SNP array (Pearson r=0.81, $p<10^{-12}$). Similarly, read counts of NGS amplicons also correlated with CN determined by SNP array, but to a lesser extent (Pearson r=0.47; $p<10^{-12}$).

**Integration of NGS *allelic ratios* and *read counts* into single calls of chromosomal alterations**

Using SNP arrays, combining copy number and allelic imbalance into a single analysis can (1) identify and discriminate normal chromosomes from chromosomes with copy number alterations or and/or loss of heterozygosity, (2) estimate the proportion of tumor cells with a chromosomal alteration, with applications for determining the clonality and the proportion of normal cells in a tumor sample, and (3) determine tumor ploidy [39,43] (Figure 1C; Figure 3C).

From the method exposed above (example 1 - section D) the inventors tested if it is possible to deliver such information from targeted NGS.

Scatterplots of SNPs with *allelic ratios* and *read counts* generated by NGS showed distinct clusters of SNPs, corresponding to distinct chromosomal statuses. An instance is provided Figure 1C and 1D, showing a specific type of chromosome alterations (chromosome losses), the proportion of tumor cells (~85%), and a subclonal events (additional chromosome losses in 40% of cells). Tumor tetraploidy can also be deduced.Figure 3C and D).

An automated detection of chromosomal gains and losses was implemented in TARGOMICs. An original combination of *allelic ratios* and *read counts* scatterplots was used, this combination considered the better reliability of *allelic ratio* compared to *read counts* (Figure 3A). A restricted variety of chromosomal statuses was used, including "normal", "loss" or "gain". Chromosomes loss, normal and gain were detected with sensitivity and specificity of 89, 72 and 31%, and 81, 93, 98% respectively, using SNP arrays as a gold standard (Figure 3B, 3C and 3D, Table 6). Of note, 38% of false positive chromosome losses and 26% of false negative normal diploid heterozygous chromosomes correspond to a subset of chromosomic regions with obvious loss of heterozygosity (Figure 3B and 3C), suggesting focal events detected by NGS at the gene level, but skipped by SNP arrays at the chromosome level.

In the validation cohort of 449 tumors, TARGOMICs performance was comparable, with chromosomes loss, normal and gain detected with sensitivity and specificity of 87, 80 and 23%, and 83, 78, 96% respectively, using SNP arrays as a gold standard (Figure 3E, 3F and 3G).

An automated detection of gene homozygous deletion and high level gene amplification was also implemented, based on *read counts* only. 18/22 homozygous deletions were detected (sensitivity: 82% ; specificity of 100% ; Figure 5, Table 4).

**Assessment of DNA methylation by targeted NGS**

Tumoral methylation status is often evaluated for CpG islands in gene promoter regions. Determining methylation status by NGS is challenging for several reasons. First sequences are repetitive (CG > 50%). Moreover these genomic regions commonly display stretches of homopolymers. The latter are responsible for false positive indels, especially when using semiconductor targeted NGS. In addition, when determining DNA methylation status by bisulfite treatment and NGS, unmethylated cytosins are transformed into uracyls (then thymins after PCR), whereas methylated cytosins remain cytosins. Thereafter, cytosins become rare, and the 4-bases genetic code becomes a 3-bases code. For these reasons, standard aligners do not perform well.

90 CpG islands (15 distinct islands from 12 samples of adrenocortical carcinomas) were analyzed. Using BISMARK [41], an aligner specifically developed for NGS after bisulfite, the number of sequences properly aligned was low, with a median coverage depth of 635 reads per CpG despite high starting number of reads (median: 137,484 reads per sample for 8 CpG islands; range: 84,075 to 560,494; Figure 4A). In this context, whether getting rid of homopolymers could increase the alignment performance was tested. After homopolymer compression (and their common false positive indels), coverage depth was not increased, with a median of 528 reads per CpG (Student p=0.13; Figure 4A).

BISMARK alignment (using bowtie [44]) is based on multiple seeding all over reference sequences. The inventors get the idea of testing wether restricting the alignment seeding to primers 3' end to increase coverage. This original alignment technique was implemented in TARGOMICs. Using TARGOMICs, median coverage depth significantly increased to 3990 reads per CpG (Student $p<10^{-12}$; Figure 4A). Of note proportion of methylated allele counted for each CpG by BISMARK and TARGOMICs remains highly correlated (Pearson r=0.86, $p<10^{-16}$; Figure 4B), thus validating TARGOMICs.

For 6 tumors, pangenomic methylation array were performed. These tumors were classified as CpG methylator phenotype (CIMP) high (N=2), CIMP intermediate (N=2), or non-CIMP (N=2) depending on the global level of methylation in CpG islands. Targeted methylation measurements confirmed this classification, with proportions of methylated CpGs significantly higher in CIMP-high and CIMP-intermediate compared to non-CIMP (paired Student p=0.049 and 0.11 respectively; Figure 4C). Using these 6 tumors as a training set, within the CpG islands, the CpGs, with a significant and positive correlation between the global tumor methylation level measured by methylation array, and the methylation measured by TARGOMICs were looked for. 22 CpGs from 4 islands have been identified (not shown).

The performance of methylation measurements by the method of the Inventors (so called TARGOMICs) was confirmed in an extended cohort of 20 additional adrenocortical carcinomas, using the 22 selected CpGs. Proportions of methylated CpGs measured by NGS strongly correlated with methylation status measured by MS-MLPA (not shown).

**Conclusion**

Inventors have developped a targeted NGS method to detect chromosomal alterations and DNA methylation status, in addition to calling mutations using an original algorithm. Combining such independent information into a single analysis should improve tumor classification for each patient. This opens the way to fully exploit in clinical routine the recent molecular discoveries arisen from massive pangenomic analyses.

Performance of NGS for calling chromosomal alterations was assessed against SNP arrays. Especially SNP arrays were used to generate DNA copy number and allelic ratios for entire chromosome arms. Considering entire chromosome arms instead of gene regions warrants a robust and sensitive detection chromosome alterations by SNP array. Indeed, targeted NGS method developped by the inventors is even able to properly detect losses of one DNA copy, provided *allelic ratios* of heterozygous SNPs are considered.

In oncogenetic, DNA methyl status is important for prognosis and potentially for treatment orientation. CpG island hypermethylation is a well-known mechanism of tumor suppressor [47]. Inventors also developed a pipeline optimized for using targeted NGS for calling methylation status which is fully integrated within the developped targeted NGS method of the invention. In terms of experimental procedure, this requires adding a second sequencing library (a single pool library from bisulfite treated DNA), to the first two-pools library required for standard targeted NGS panel and heterozygous SNPs. Including methylation analysis in the same NGS experiment provides a particular advantage over the other techniques performing methylation analyses, such as pyrosequencing MS-MLPA or MEDIPseq [48]. Indeed, it does not increase much the time of NGS experiment, all results are available at once, no extra equipment is required, and bisulfite treatment and PCR are easy to handle. The cost of an extra-library is also limited, especially since a limited sequencing depth is sufficient. In addition, using the algorithm developped by the inventors (TARGOMICs), the alignment efficiency has been increased more than 5 times compared to BISMARK [41], a commonly used aligner and methylation caller.

## References listing

1. Zheng S, Cherniack AD, Dewal N, Moffitt RA, Danilova L, Murray BA, Lerario AM, Else T, Knijnenburg TA, Ciriello G, Kim S, Assie G, Morozova O, Akbani R, Shih J, Hoadley KA, Choueiri TK, Waldmann J, Mete O, Robertson AG, Wu H-T, Raphael BJ, Shao L, Meyerson M, Demeure MJ, Beuschlein F, Gill AJ, Sidhu SB, Almeida MQ, Fragoso MCBV, Cope LM, Kebebew E, Habra MA, Whitsett TG, Bussey KJ, Rainey WE, Asa SL, Bertherat J, Fassnacht M, Wheeler DA, Hammer GD, Giordano TJ, Verhaak RGW: Comprehensive Pan-Genomic Characterization of Adrenocortical Carcinoma. Cancer Cell 2016, 29:723-736.
2. Hrdlickova R, Toloue M, Tian B: RNA-Seq methods for transcriptome analysis: RNA-Seq. Wiley Interdisciplinary Reviews: RNA [Internet] 2016 [cited 2016 Jun 24], . Available from: http://doi.wiley.com/10.1002/wrna.1364
3. Tomczak K, Czerwinska P, Wiznerowicz M: Review The Cancer Genome Atlas (TCGA): an immeasurable source of knowledge. Wspó czesna Onkologia 2015, 1A:68-77.
4. Hausser J, Zavolan M: Identification and consequences of miRNA-target interactions - beyond repression of gene expression. Nature Reviews Genetics 2014, 15:599-612.
5. Russo CD, Di Giacomo G, Cignini P, Padula F, Mangiafico L, Mesoraca A, D'Emidio L, McCluskey MR, Paganelli A, Giorlandino C: Comparative study of aCGH and Next Generation Sequencing (NGS) for chromosomal microdeletion and microduplication screening. Journal of prenatal medicine 2014, 8:57.
6. Abel HJ, Duncavage EJ: Detection of structural DNA variation from next generation sequencing data: a review of informatic approaches. Cancer Genetics 2013, 206:432-440.
7. Assié G, Jouinot A, Bertherat J: The "omics" of adrenocortical tumours for personalized medicine. Nature Reviews Endocrinology 2014, 10:215-228.
8. Weisenberger DJ: Characterizing DNA methylation alterations from The Cancer Genome Atlas. Journal of Clinical Investigation 2014, 124:17-23.
9. Shull AY, Noonepalle SK, Lee E-J, Choi J-H, Shi H: Sequencing the Cancer Methylome. In: Verma M, editor. Cancer Epigenetics [Internet], New York, NY, Springer New York, 2015 [cited 2016 Jun 24], pp. 627-651. Available from: http://link.springer.com/10.1007/978-1-4939-1804-1_33
10. Sonnet M, Baer C, Rehli M, Weichenhan D, Plass C: Enrichment of methylated DNA by methyl-CpG immunoprecipitation. Methods Mol Biol 2013, 971:201-212.
11. Offit K: Decade in review-genomics: A decade of discovery in cancer genomics. Nature Reviews Clinical Oncology 2014, 11:632-634.
12. Cline MS, Craft B, Swatloski T, Goldman M, Ma S, Haussler D, Zhu J: Exploring TCGA Pan-Cancer Data at the UCSC Cancer Genomics Browser. Scientific Reports [Internet] 2013 [cited 2016 Jun 24], 3. Available from: http://www.nature.com/articles/srep02652
13. Zhao Q, Shi X, Xie Y, Huang J, Shia B, Ma S: Combining multidimensional genomic measurements for predicting cancer prognosis: observations from TCGA. Briefings in Bioinformatics 2015, 16:291-303.
14. Neapolitan R, Horvath CM, Jiang X: Pan-cancer analysis of TCGA data reveals notable signaling pathways.

BMC Cancer [Internet] 2015 [cited 2016 Jun 13], 15. Available from: http://www.biomedcentral.com/1471-2407/15/516
15. Nakagawa H, Wardell CP, Furuta M, Taniguchi H, Fujimoto A: Cancer whole-genome sequencing: present and future. Oncogene 2015, 34:5943-5950.
16. Ching T, Peplowska K, Huang S, Zhu X, Shen Y, Molnar J, Yu H, Tiirikainen M, Fogelgren B, Fan R, Garmire LX: Pan-Cancer Analyses Reveal Long Intergenic Non-Coding RNAs Relevant to Tumor Diagnosis, Subtyping and Prognosis. EBioMedicine 2016, 7:62-72.
17. Vockley JG, Niederhuber JE: Diagnosis and treatment of cancer using genomics. BMJ 2015, 350:h1832-h1832.
18. Wijesinghe P, Bollig-Fischer A: Lung Cancer Genomics in the Era of Accelerated Targeted Drug Development. In: Ahmad A, Gadgeel SM, editors. Lung Cancer and Personalized Medicine: Novel Therapies and Clinical Management [Internet], Cham, Springer International Publishing, 2016 [cited 2016 Jun 24], pp. 1-23. Available from: http://link.springer.com/10.1007/978-3-319-24932-2_1
19. Tang B, Hsu P-Y, Huang TH-M, Jin VX: Cancer omics: From regulatory networks to clinical outcomes. Cancer Letters 2013, 340:277-283.
20. Desai A, Jere A: Next-generation sequencing: ready for the clinics? Clinical Genetics 2012, 81:503-510.
21. Xuan J, Yu Y, Qing T, Guo L, Shi L: Next-generation sequencing in the clinic: Promises and challenges. Cancer Letters 2013, 340:284-295.
22. Dietel M, Jöhrens K, Laffert MV, Hummel M, Bläker H, Pfitzner BM, Lehmann A, Denkert C, Darb-Esfahani S, Lenze D, others: A 2015 update on predictive molecular pathology and its role in targeted cancer therapy: a review focussing on clinical relevance. Cancer Gene Therapy 2015, 22:417-430.
23. Sikkema-Raddatz B, Johansson LF, de Boer EN, Almomani R, Boven LG, van den Berg MP, van Spaendonck-Zwarts KY, van Tintelen JP, Sijmons RH, Jongbloed JDH, Sinke RJ: Targeted Next-Generation Sequencing can Replace Sanger Sequencing in Clinical Diagnostics. Human Mutation 2013, 34:1035-1042.
24. Shao D, Lin Y, Liu J, Wan L, Liu Z, Cheng S, Fei L, Deng R, Wang J, Chen X, Liu L, Gu X, Liang W, He P, Wang J, Ye M, He J: A targeted next-generation sequencing method for identifying clinically relevant mutation profiles in lung adenocarcinoma. Scientific Reports 2016, 6:22338.
25. Devarajan B, Prakash L, Kannan TR, Abraham AA, Kim U, Muthukkaruppan V, Vanniarajan A: Targeted next generation sequencing of RB1 gene for the molecular diagnosis of Retinoblastoma. BMC Cancer [Internet] 2015 [cited 2016 Jun 17], 15. Available from: http://www.biomedcentral.com/1471-2407/15/320
26. Boonham N, Kreuze J, Winter S, van der Vlugt R, Bergervoet J, Tomlinson J, Mumford R: Methods in virus diagnostics: From ELISA to next generation sequencing. Virus Research 2014, 186:20-31.
27. Pak TR, Kasarskis A: How Next-Generation Sequencing and Multiscale Data Analysis Will Transform Infectious Disease Management. Clinical Infectious Diseases 2015, :civ670.
28. Barzon L, Lavezzo E, Militello V, Toppo S, Palù G: Applications of Next-Generation Sequencing Technologies to Diagnostic Virology. International Journal of Molecular Sciences 2011, 12:7861-7884.
29. Shen R, Seshan VE: FACETS: allele-specific copy number and clonal heterogeneity analysis tool for high-throughput DNA sequencing. Nucleic Acids Research 2016, :gkw520.
30. Johansson LF, van Dijk F, de Boer EN, van Dijk-Bos KK, Jongbloed JDH, van der Hout AH, Westers H, Sinke RJ, Swertz MA, Sijmons RH, Sikkema-Raddatz B: CoNVaDING: Single Exon Variation Detection in Targeted NGS Data. Human Mutation 2016, 37:457-464.
31. Boeva V, Popova T, Bleakley K, Chiche P, Cappo J, Schleiermacher G, Janoueix-Lerosey I, Delattre O, Barillot E: Control-FREEC: a tool for assessing copy number and allelic content using next-generation sequencing data. Bioinformatics 2012, 28:423-425.
32. Duan J, Zhang J-G, Deng H-W, Wang Y-P: CNV-TV: A robust method to discover copy number variation from short sequencing reads. BMC Bioinformatics 2013, 14:150.
33. Zhao X, Wang A, Walter V, Patel NM, Eberhard DA, Hayward MC, Salazar AH, Jo H, Soloway MG, Wilkerson MD, Parker JS, Yin X, Zhang G, Siegel MB, Rosson GB, Earp HS, Sharpless NE, Gulley ML, Weck KE, Hayes DN, Moschos SJ: Combined Targeted DNA Sequencing in Non-Small Cell Lung Cancer (NSCLC) Using UNCseq and NGScopy, and RNA Sequencing Using UNCqeR for the Detection of Genetic Aberrations in NSCLC. Calogero RA, editor. PLOS ONE 2015, 10:e0129280.
34. Winchester L, Yau C, Ragoussis J: Comparing CNV detection methods for SNP arrays. Briefings in Functional Genomics and Proteomics 2009, 8:353-366.
35. Zhang X, Du R, Li S, Zhang F, Jin L, Wang H: Evaluation of copy number variation detection for a SNP array platform. BMC bioinformatics 2014, 15:1.
36. Zhang D, Qian Y, Akula N, Alliey-Rodriguez N, Tang J, Gershon ES, Liu C, others: Accuracy of CNV detection from GWAS data. PLoS One 2011, 6:e14511.
37. Barreau O, de Reynies A, Wilmot-Roussel H, Guillaud-Bataille M, Auzan C, René-Corail F, Tissier F, Dousset B, Bertagna X, Bertherat J, Clauser E, Assié G: Clinical and Pathophysiological Implications of Chromosomal Alterations in Adrenocortical Tumors: An Integrated Genomic Approach. The Journal of Clinical Endocrinology & Metabolism 2012, 97:E301-E311.

38. Assié G, Letouzé E, Fassnacht M, Jouinot A, Luscap W, Barreau O, Omeiri H, Rodriguez S, Perlemoine K, René-Corail F, Elarouci N, Sbiera S, Kroiss M, Allolio B, Waldmann J, Quinkler M, Mannelli M, Mantero F, Papathomas T, De Krijger R, Tabarin A, Kerlan V, Baudin E, Tissier F, Dousset B, Groussin L, Amar L, Clauser E, Bertagna X, Ragazzon B, Beuschlein F, Libé R, de Reyniès A, Bertherat J: Integrated genomic characterization of adrenocortical carcinoma. Nature Genetics 2014, 46:607-612.

39. Popova T, Manié E, Stoppa-Lyonnet D, Rigaill G, Barillot E, Stern MH: Genome Alteration Print (GAP): a tool to visualize and mine complex cancer genomic profiles obtained by SNP arrays. Genome biology 2009, 10:1.

40. Li L-C, Dahiya R: MethPrimer: designing primers for methylation PCRs. Bioinformatics 2002, :1427-1431.

41. Krueger F, Andrews SR: Bismark: a flexible aligner and methylation caller for Bisulfite-Seq applications. Bioinformatics 2011, 27:1571-1572.

42. Tariq K, Ghias K: Colorectal cancer carcinogenesis: a review of mechanisms. Cancer biology & medicine 2016, 13:120.

43. Assié G, LaFramboise T, Platzer P, Bertherat J, Stratakis CA, Eng C: SNP arrays in heterogeneous tissue: highly accurate collection of both germline and somatic genetic information from unpaired single tumor samples. Am J Hum Genet 2008, 82:903-915.

44. Langmead B, Salzberg SL: Fast gapped-read alignment with Bowtie 2. Nat Methods 2012, 9:357-359.

45. Harrison CJ: Targeting signaling pathways in acute lymphoblastic leukemia: new insights. Hematology Am Soc Hematol Educ Program 2013, 2013:118-125.

46. Moalic-Allain V, Mercier B, Gueguen P, Ferec C: Next generation sequencing with a semi-conductor technology (Ion Torrent PGM™) for HLA typing: overall workflow performance and debate. Ann Biol Clin (Paris) 2016, 74:449-456.

47. Witte T, Plass C, Gerhauser C: Pan-cancer patterns of DNA methylation. Genome Med 2014, 6:66.

48. Jeong HM, Lee S, Chae H, Kim R, Kwon MJ, Oh E, Choi Y-L, Kim S, Shin YK: Efficiency of methylated DNA immunoprecipitation bisulphite sequencing for whole-genome DNA methylation analysis. Epigenomics 2016, 8:1061-1077.

49. Gicquel C, Bertagna X, Gaston V, Coste J, Louvel A, Baudin E, Bertherat J, Chapuis Y, Duclos JM, Schlumberger M, Plouin PF, Luton JP, Le Bouc Y. Molecular markers and long-term recurrences in a large cohort of patients with sporadic adrenocortical tumors. Cancer Res. 2001, 61(18): 6762-6767.

50. Le Tourneau C, Delord J-P, Gonçalves A, Gavoille C, Dubot C, Isambert N, Campone M, Trédan O, Massiani M-A, Mauborgne C, Armanet S, Servant N, Bièche I, Bernard V, Gentien D, Jezequel P, Attignon V, Boyault S, Vincent-Salomon A, Servois V, Sablin M-P, Kamal M, Paoletti X, SHIVA investigators: Molecularly targeted therapy based on tumour molecular profiling versus conventional therapy for advanced cancer (SHIVA): a multicentre, open-label, proof-of-concept, randomised, controlled phase 2 trial. Lancet Oncol 2015, 16:1324-1334.

51. Jouinot A, Assie G, Libe R, Fassnacht M, Papathomas T, Barreau O, de la Villeon B, Faillot S, Hamzaoui N, Neou M, Perlemoine K, Rene-Corail F, Rodriguez S, Sibony M, Tissier F, Dousset B, Sbiera S, Ronchi C, Kroiss M, Korpershoek E, de Krijger R, Waldmann J, K D, Bartsch, Quinkler M, Haissaguerre M, Tabarin A, Chabre O, Sturm N, Luconi M, Mantero F, Mannelli M, Cohen R, Kerlan V, Touraine P, Barrande G, Groussin L, Bertagna X, Baudin E, Amar L, Beuschlein F, Clauser E, Coste J, Bertherat J: DNA Methylation Is an Independent Prognostic Marker of Survival in Adrenocortical Cancer. J Clin Endocrinol Metab 2017, 102:923-932.

## Claims

1. A Next-Generation DNA Sequencing (NGS) method of analysing a cancer of a patient comprising the detection, in a sample of said patient, of:

   - at least one characteristic alteration of chromosome regions identified for said cancer from a set of genes from these regions,
   - specific gene mutations or at least one characteristic pattern of mutations in a set of genes identified in said cancer, and
   - at least one characteristic pattern of DNA methylation status of chromosome regions identified as having an altered methylation status in said cancer,

   all these three detection steps being implemented in a single NGS design

2. The NGS method according to claim 1, wherein the detection step of detecting at least one characteristic alteration of chromosome regions comprises identifying homozygous deletions and loss of heterozygosity (LOH) within the set of genes of said chromosome regions identified for said cancer.

3. The NGS method according to the preceding claim, wherein the detection step of detecting at least one characteristic alteration of chromosome regions further comprises analysing at least 5 SNPs per chromosome arm of interest for searching heterozygous deletions or LOH (Loss of Heterozygosity), said at least 5 SNPs being known to be highly heterozygous in the general population.

4. The NGS method according to any of claims 2 and 3, wherein the step of detecting at least one characteristic alteration of chromosome regions is performing by a combined analysis of the allelic ratio and the amplicons read counts.

5. The NGS method according to any of claims 1 to 4, wherein the step of detecting at least one specific pattern of DNA methylation status is carried out onto bisulfite-treated DNA.

6. The NGS method according to claim 5, wherein the detection of at least one specific pattern of DNA methylation comprises the analysis of the methylation status of the CpG islands.

7. The NGS method according to claim 5 or 6, wherein the alignment of sequences of NGS methylation analysis is operated (i) after a step of replacing the stretches of identical bases by only one corresponding base, except around the CpGs, the dinucleotides CG, TG and CA being excluded from this compression and (ii) wherein the alignment over the reference sequence is restricted to the use of 3' primers end.

8. The NGS method according to any of the preceding claims, wherein analysing the cancer of a patient comprises the assignment of said patient to a specific group of patients corresponding to a specific molecular type of tumor.

9. The NGS method according to the preceding claim, wherein the assignment of said patient to a specific group of patients is used to prognose the response of said patient to a treatment, or to prognose the survival time of said patient.

10. The NGS method according to claim 1, wherein the cancer is selected in the group comprising adrenocortical carcinoma, breast cancer, colorectal cancer, ovarian cancer, lung cancer, pancreatic cancer, sarcoma, urothelial cancer, head and neck squamous cell carcinoma, adenoma carcinoma with unknown primitive tumor, endometrial cancer, cervical cancer, oesogastric cancer, adenoid cystic carcinoma, cholangiocarcinoma, neuroendocrine tumor, melanoma, anal squamous cell carcinoma, kidney cancer, uveal melanoma, germline tumor, hepatocellular carcinoma, parotid cancer, thyroid cancer, undifferentiated nasopharyngeal cancer of the cavum, Merkel cell carcinoma, mesothelioma, penile squamous cell carcinoma, peritoneal cancer, chemodectoma, corticosurrenaloma, desmoid tumor, epithelioid hemangiocarcinoma, meningioma, midline carcinoma, mixopapillary ependymoma, non-adenoid cystic carcinoma salivary gland tumor, ocular adenocarcinoma, pelvic squamous cell carcinoma, pleiomorphic carcinoma of the tongue, prostate adenocarcinoma, thymic cancer, and squamous cell carcinoma of the vulva.

11. The NGS method according to claim 10, wherein the cancer is an adrenocortical carcinoma and:

   - the chromosome regions identified as bearing at least one characteristic patterns of alteration and at least one characteristic pattern of mutations for said cancer are the regions comprising at least one of the genes selected from *HSD3B2, CTNNB1*,*APC, CYP21A2, DAXX, BAI1*, *CDKN2A, CYP17A1, MEN1, MCAM, RB1, TP53, RNF43, ZNRF3, MED12* and CDK4.
- the chromosome regions identified as having an altered methylation status comprise at least one of CpG region selected from Cg07384961, Cg14021073, Cg21494776, Cg23130254, Cg20312228, Cg01635061, Cg27234090, Cg04582938, Cg06039392, Cg10167296, Cg10743104, Cg27425675, Cg16689634, Cg01120165 and Cg15284635.

**12.** A kit comprising a single NGS design for analysing (i) specific alterations of chromosome regions, (ii) specific gene mutations, and (iii) DNA methylation status of specific chromosome regions.

**13.** A kit according to claim 12, comprising a NGS design comprising one or more primer sets corresponding to at least one gene selected from, ZNRF3, TP53, RB1, CDKN2A, CDK4.

**14.** A kit according to claim 12, comprising a NGS design comprising one or more primer sets corresponding to at least one CpG island identified selected from Cg07384961, Cg14021073, Cg21494776 and Cg23130254.

**15.** Use of a kit according to claims 13 and/or 14 for analysing adrenocortical carcinoma.

**16.** A NGS method for sequencing CpG islands comprising the steps of:

i) replacing the stretches of identical bases by only one base, except around the CpGs, the dinucleotides CG, TG and CA being excluded from this compression, and
ii) performing the alignment of sequences by restricting the alignment seeding to the 3’ end of each primer.

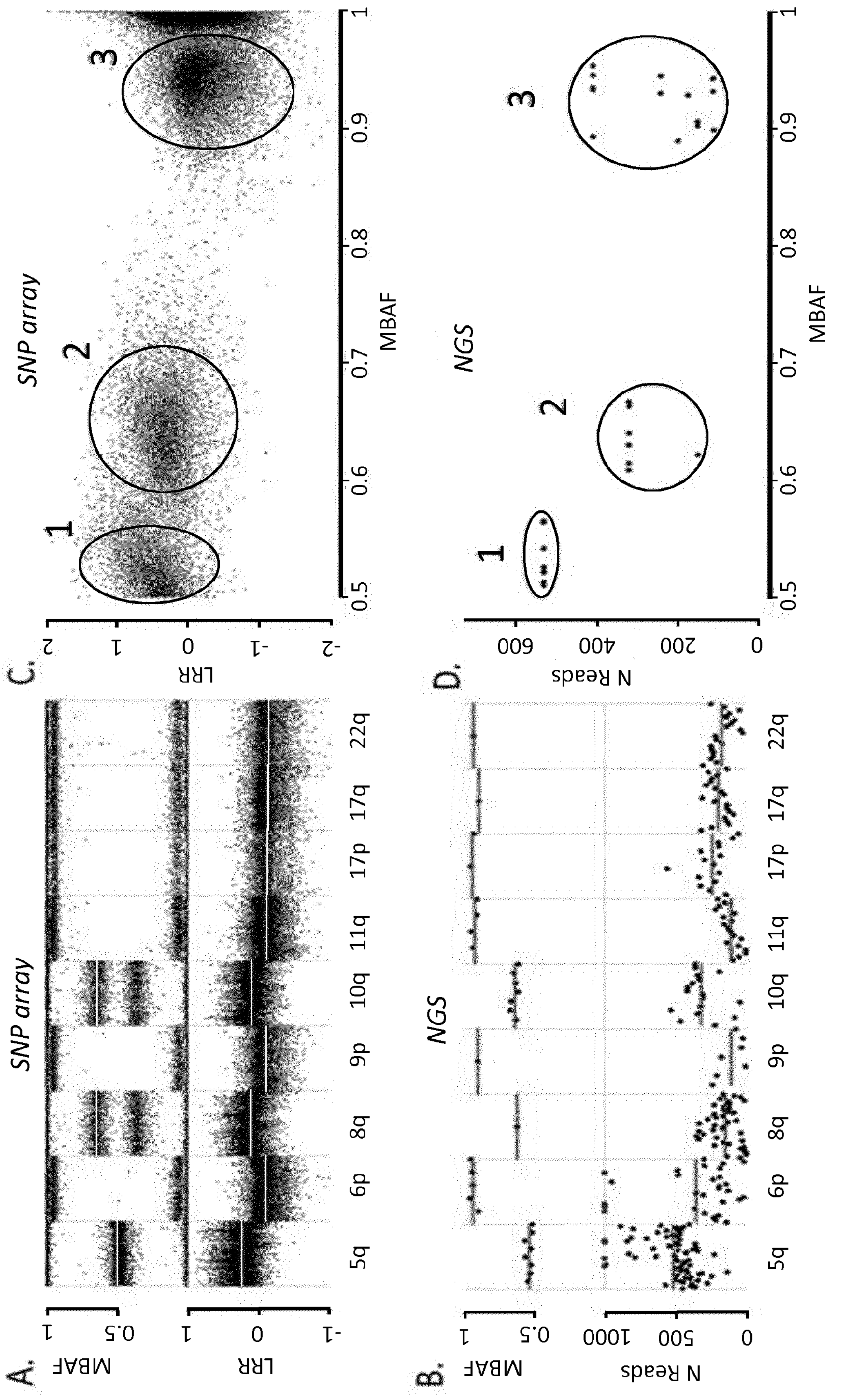
A.
SNP array
MBAF
LRR
5q
6p
8q
9p
10q
11q
17p
17q
22q
B.
NGS
MBAF
N Reads
C.
SNP array
LRR
MBAF
1
2
3
D.
NGS
N Reads
MBAF
1
2
3

FIGURE 1

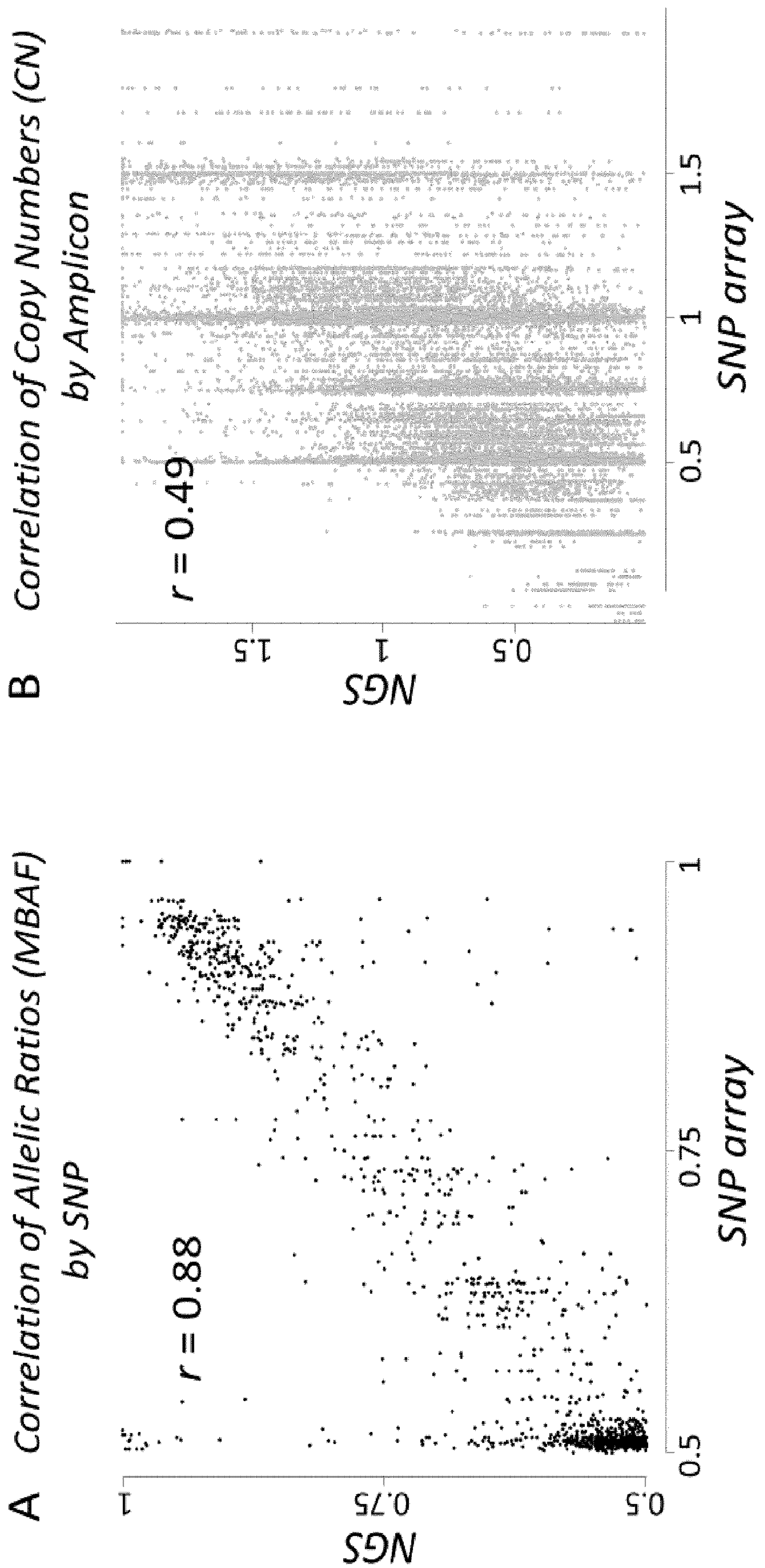
A
Correlation of Allelic Ratios (MBAF) by SNP
r = 0.88
NGS
0.5
0.75
1
SNP array
0.5
0.75
1
B
Correlation of Copy Numbers (CN) by Amplicon
r = 0.49
NGS
0.5
1
1.5
SNP array
0.5
1
1.5

FIGURE 2

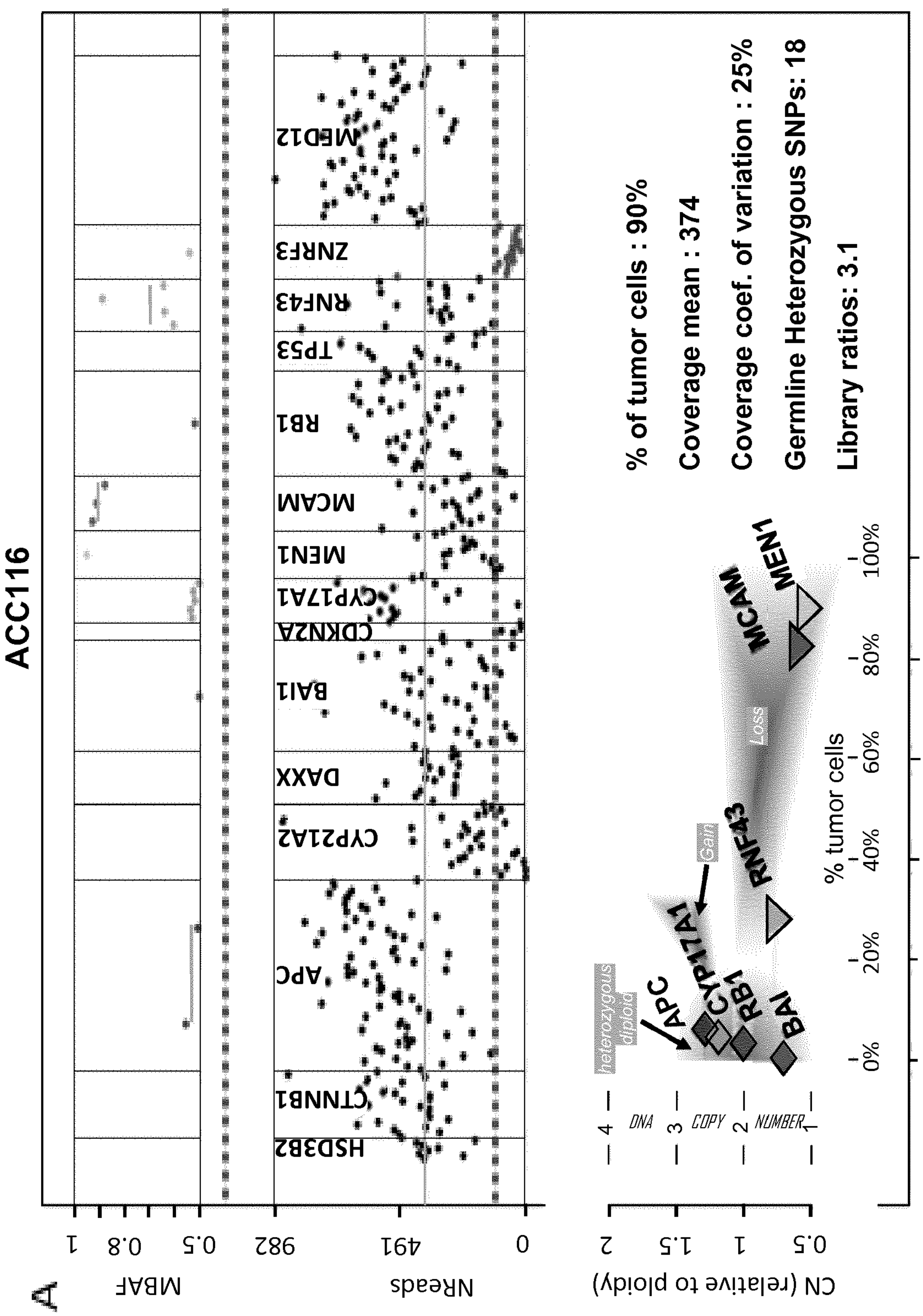
A
ACC116
MBAF
NReads
CN (relative to ploidy)
HSD3B2
CTNNB1
APC
CYP21A2
DAXX
BAI1
CDKN2A
CYP17A1
MEN1
MCAM
RB1
TP53
RNF43
ZNRF3
MED12
% of tumor cells : 90%
Coverage mean : 374
Coverage coef. of variation : 25%
Germline Heterozygous SNPs: 18
Library ratios: 3.1
heterozygous diploid
Gain
Loss
DNA COPY NUMBER
% tumor cells
MBAF

FIGURE 3

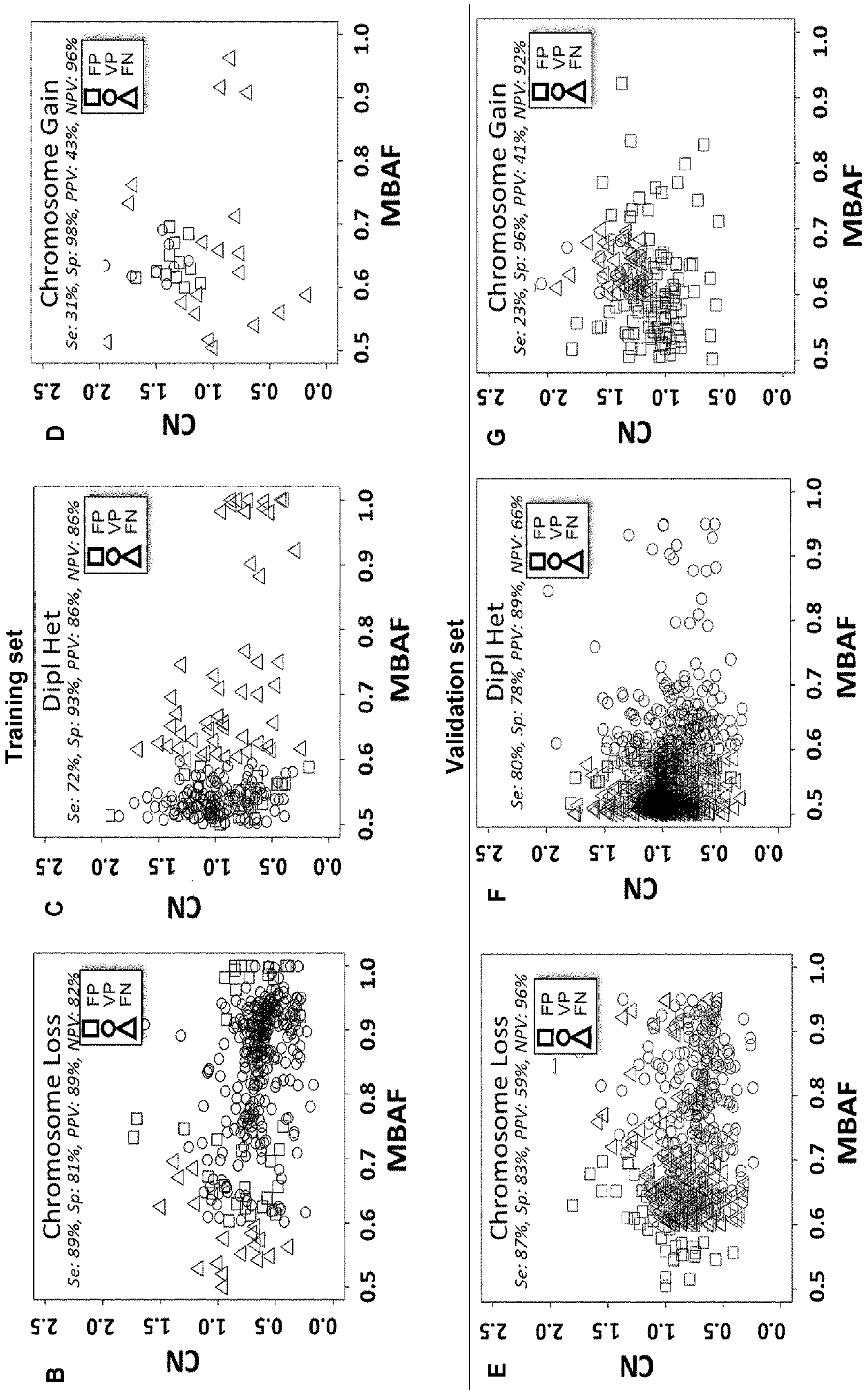


FIGURE 3 (continuation and end)

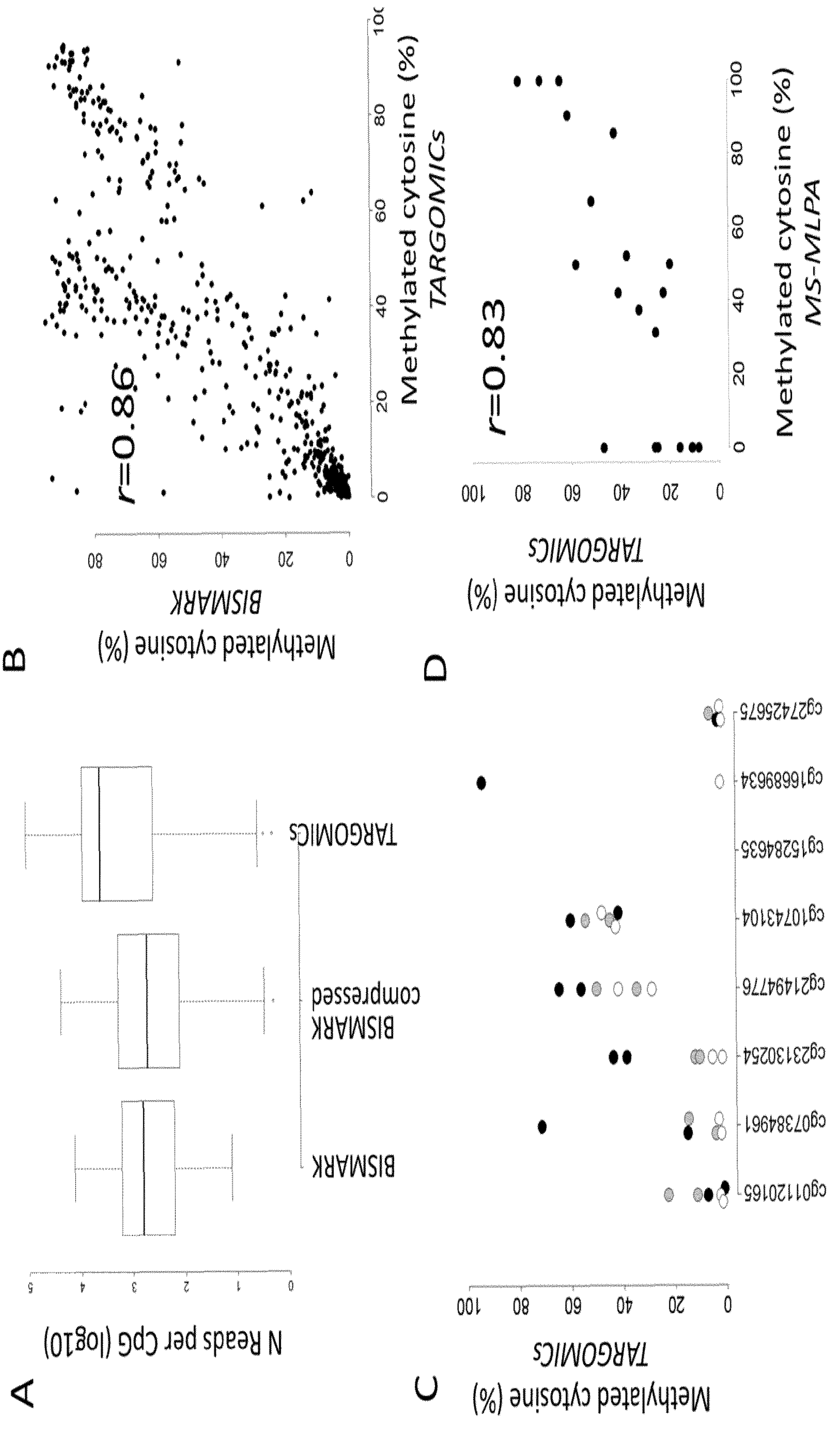
A
N Reads per CpG (log10)
BISMARK
BISMARK compressed
TARGOMICs
B
Methylated cytosine (%) BISMARK
r=0.86
Methylated cytosine (%) TARGOMICs
C
Methylated cytosine (%) TARGOMICs
cg01120165
cg07384961
cg23130254
cg21494776
cg10743104
cg15284635
cg16689634
cg27425675
D
Methylated cytosine (%) TARGOMICs
r=0.83
Methylated cytosine (%) MS-MLPA

FIGURE 4

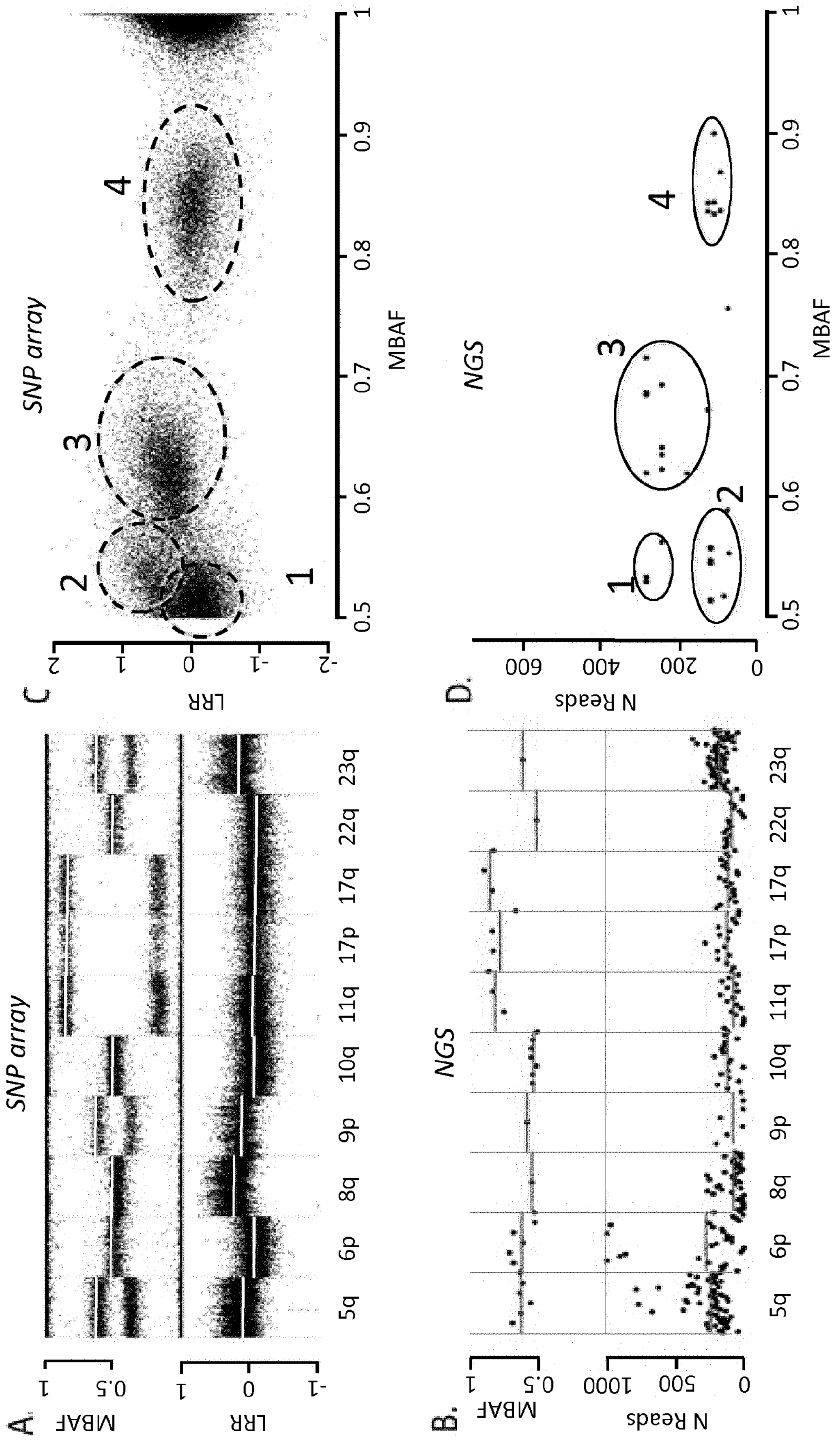

FIGURE 5

| | Amplicon | Primer Pool | Chromosome | Start_Position_Hg19 | End_Position_Hg19 | Gene Symbol | Number of G and C |
|---|---|---|---|---|---|---|---|
| 1 | AMPL2752422951 | 1 | 1 | 119958027 | 119958206 | HSD3B2 | 95 |
| 2 | AMPL2751117291 | 2 | 1 | 119961935 | 119962069 | HSD3B2 | 62 |
| 3 | AMPL3662733841 | 1 | 1 | 119962070 | 119962236 | HSD3B2 | 81 |
| 4 | AMPL3662733959 | 2 | 1 | 119964392 | 119964548 | HSD3B2 | 85 |
| 5 | AMPL3662757044 | 1 | 1 | 119964544 | 119964653 | HSD3B2 | 56 |
| 6 | AMPL3662763351 | 2 | 1 | 119964644 | 119964790 | HSD3B2 | 65 |
| 7 | AMPL3662774813 | 1 | 1 | 119964791 | 119964967 | HSD3B2 | 93 |
| 8 | AMPL3662792666 | 2 | 1 | 119964965 | 119965092 | HSD3B2 | 62 |
| 9 | AMPL3662813598 | 1 | 1 | 119965088 | 119965259 | HSD3B2 | 87 |
| 10 | AMPL393580014 | 1 | 3 | 41265452 | 41265617 | CTNNB1 | 55 |
| 11 | AMPL396195789 | 2 | 3 | 41265916 | 41266084 | CTNNB1 | 57 |
| 12 | AMPL686600530 | 1 | 3 | 41266085 | 41266259 | CTNNB1 | 82 |
| 13 | AMPL393871206 | 2 | 3 | 41266423 | 41266538 | CTNNB1 | 53 |
| 14 | AMPL393928487 | 1 | 3 | 41266539 | 41266706 | CTNNB1 | 74 |
| 15 | AMPL393781900 | 2 | 3 | 41266779 | 41266911 | CTNNB1 | 56 |
| 16 | AMPL393811409 | 1 | 3 | 41266912 | 41267068 | CTNNB1 | 75 |
| 17 | AMPL396157755 | 2 | 3 | 41267032 | 41267204 | CTNNB1 | 69 |
| 18 | AMPL393798925 | 1 | 3 | 41267205 | 41267365 | CTNNB1 | 63 |
| 19 | AMPL2601487334 | 2 | 3 | 41268612 | 41268786 | CTNNB1 | 62 |
| 20 | AMPL2601494879 | 1 | 3 | 41268787 | 41268863 | CTNNB1 | 32 |
| 21 | AMPL393908314 | 2 | 3 | 41274801 | 41274967 | CTNNB1 | 69 |
| 22 | AMPL393858321 | 1 | 3 | 41274975 | 41275155 | CTNNB1 | 76 |
| 23 | AMPL393926963 | 2 | 3 | 41275156 | 41275295 | CTNNB1 | 78 |
| 24 | AMPL2601504686 | 1 | 3 | 41275296 | 41275392 | CTNNB1 | 41 |
| 25 | AMPL395443475 | 2 | 3 | 41275525 | 41275636 | CTNNB1 | 40 |
| 26 | AMPL393939548 | 1 | 3 | 41275637 | 41275804 | CTNNB1 | 84 |
| 27 | AMPL393590457 | 2 | 3 | 41277191 | 41277308 | CTNNB1 | 57 |
| 28 | AMPL418552033 | 1 | 3 | 41277309 | 41277481 | CTNNB1 | 51 |
| 29 | AMPL424554115 | 2 | 3 | 41277690 | 41277846 | CTNNB1 | 60 |
| 30 | AMPL393492914 | 1 | 3 | 41277847 | 41277999 | CTNNB1 | 73 |
| 31 | AMPL393900371 | 2 | 3 | 41278057 | 41278224 | CTNNB1 | 76 |
| 32 | AMPL393788809 | 1 | 3 | 41279493 | 41279617 | CTNNB1 | 54 |
| 33 | AMPL393584967 | 2 | 3 | 41280555 | 41280733 | CTNNB1 | 89 |
| 34 | AMPL393637671 | 1 | 3 | 41280734 | 41280884 | CTNNB1 | 72 |
| 35 | AMPL393540352 | 1 | 5 | 112043355 | 112043463 | APC | 72 |
| 36 | AMPL393547046 | 2 | 5 | 112043463 | 112043586 | APC | 86 |
| 37 | AMPL394025559 | 1 | 5 | 112090527 | 112090611 | APC | 31 |
| 38 | AMPL394029673 | 2 | 5 | 112090612 | 112090764 | APC | 52 |
| 39 | AMPL708055068 | 1 | 5 | 112101931 | 112102093 | APC | 47 |
| 40 | AMPL393799322 | 2 | 5 | 112102094 | 112102164 | APC | 18 |
| 41 | AMPL393522310 | 1 | 5 | 112102863 | 112102935 | APC | 23 |
| 42 | AMPL393526133 | 2 | 5 | 112102936 | 112103106 | APC | 73 |
| 43 | AMPL396044271 | 1 | 5 | 112111245 | 112111355 | APC | 33 |
| 44 | AMPL1077060375 | 2 | 5 | 112111355 | 112111441 | APC | 27 |
| 45 | AMPL396279579 | 1 | 5 | 112116385 | 112116521 | APC | 35 |
| 46 | AMPL396316032 | 2 | 5 | 112116522 | 112116691 | APC | 59 |
| 47 | AMPL3684296347 | 1 | 5 | 112128058 | 112128186 | APC | 35 |
| 48 | AMPL1073015532 | 2 | 5 | 112128182 | 112128245 | APC | 27 |
| 49 | AMPL708240616 | 1 | 5 | 112136970 | 112137128 | APC | 57 |
| 50 | AMPL674250640 | 2 | 5 | 112151129 | 112151305 | APC | 68 |
| 51 | AMPL395920440 | 1 | 5 | 112154620 | 112154782 | APC | 66 |
| 52 | AMPL2500220999 | 2 | 5 | 112154783 | 112154899 | APC | 61 |
| 53 | AMPL2500235643 | 1 | 5 | 112154900 | 112155064 | APC | 78 |
| 54 | AMPL623962662 | 2 | 5 | 112157518 | 112157622 | APC | 32 |
| 55 | AMPL395602023 | 1 | 5 | 112157623 | 112157784 | APC | 46 |
| 56 | AMPL3684330699 | 2 | 5 | 112162766 | 112162878 | APC | 45 |
| 57 | AMPL1076664229 | 1 | 5 | 112162879 | 112162955 | APC | 28 |
| 58 | AMPL393460395 | 2 | 5 | 112163604 | 112163681 | APC | 32 |
| 59 | AMPL1986084112 | 1 | 5 | 112164496 | 112164562 | APC | 15 |
| 60 | AMPL3658942647 | 2 | 5 | 112164576 | 112164726 | APC | 50 |
| 61 | AMPL394083357 | 1 | 5 | 112170546 | 112170661 | APC | 34 |
| 62 | AMPL3658946503 | 2 | 5 | 112170662 | 112170819 | APC | 66 |
| 63 | AMPL3658977123 | 1 | 5 | 112170820 | 112170892 | APC | 24 |
| 64 | AMPL393924882 | 2 | 5 | 112173229 | 112173398 | APC | 63 |
| 65 | AMPL393982786 | 1 | 5 | 112173397 | 112173557 | APC | 71 |
| 66 | AMPL394058174 | 2 | 5 | 112173558 | 112173721 | APC | 61 |
| 67 | AMPL393940080 | 1 | 5 | 112173722 | 112173878 | APC | 63 |
| 68 | AMPL394010986 | 2 | 5 | 112173879 | 112174042 | APC | 76 |
| 69 | AMPL394069549 | 1 | 5 | 112174043 | 112174215 | APC | 60 |
| 70 | AMPL1896248181 | 2 | 5 | 112174214 | 112174294 | APC | 29 |
| 71 | AMPL393939939 | 1 | 5 | 112174295 | 112174418 | APC | 45 |
| 72 | AMPL3659014914 | 2 | 5 | 112174419 | 112174570 | APC | 54 |
| 73 | AMPL3659060766 | 1 | 5 | 112174571 | 112174745 | APC | 71 |
| 74 | AMPL2021570496 | 2 | 5 | 112174746 | 112174907 | APC | 54 |
| 75 | AMPL3659131464 | 1 | 5 | 112174908 | 112174991 | APC | 37 |

FIGURE 6 (TABLE 2)

| | | | | | | | |
|---|---|---|---|---|---|---|---|
| 76 | AMPL3659144068 | 2 | 5 | 112174992 | 112175145 | APC | 57 |
| 77 | AMPL1697126918 | 1 | 5 | 112175144 | 112175276 | APC | 57 |
| 78 | AMPL1998979265 | 2 | 5 | 112175276 | 112175452 | APC | 83 |
| 79 | AMPL3659117598 | 1 | 5 | 112175453 | 112175615 | APC | 78 |
| 80 | AMPL3659187693 | 2 | 5 | 112175616 | 112175795 | APC | 78 |
| 81 | AMPL3659234509 | 1 | 5 | 112175796 | 112175921 | APC | 54 |
| 82 | AMPL394065728 | 2 | 5 | 112175922 | 112176090 | APC | 58 |
| 83 | AMPL394104630 | 1 | 5 | 112176091 | 112176216 | APC | 60 |
| 84 | AMPL393968951 | 2 | 5 | 112176217 | 112176380 | APC | 71 |
| 85 | AMPL394053752 | 1 | 5 | 112176381 | 112176495 | APC | 48 |
| 86 | AMPL707927515 | 2 | 5 | 112176496 | 112176628 | APC | 54 |
| 87 | AMPL3659125987 | 1 | 5 | 112176627 | 112176796 | APC | 52 |
| 88 | AMPL393963473 | 2 | 5 | 112176797 | 112176918 | APC | 45 |
| 89 | AMPL3659172513 | 1 | 5 | 112176918 | 112177006 | APC | 36 |
| 90 | AMPL3659190238 | 2 | 5 | 112177007 | 112177097 | APC | 38 |
| 91 | AMPL1381214529 | 1 | 5 | 112177098 | 112177273 | APC | 70 |
| 92 | AMPL3659044864 | 2 | 5 | 112177274 | 112177414 | APC | 58 |
| 93 | AMPL3659075384 | 1 | 5 | 112177415 | 112177536 | APC | 46 |
| 94 | AMPL3659109477 | 2 | 5 | 112177537 | 112177629 | APC | 35 |
| 95 | AMPL3659128454 | 1 | 5 | 112177630 | 112177794 | APC | 67 |
| 96 | AMPL1038874783 | 2 | 5 | 112177795 | 112177968 | APC | 60 |
| 97 | AMPL394005696 | 1 | 5 | 112177968 | 112178133 | APC | 79 |
| 98 | AMPL624225164 | 2 | 5 | 112178134 | 112178301 | APC | 79 |
| 99 | AMPL393649354 | 1 | 5 | 112178292 | 112178456 | APC | 67 |
| 100 | AMPL393679937 | 2 | 5 | 112178457 | 112178614 | APC | 59 |
| 101 | AMPL393703614 | 1 | 5 | 112178615 | 112178756 | APC | 62 |
| 102 | AMPL393635118 | 2 | 5 | 112178757 | 112178933 | APC | 81 |
| 103 | AMPL393700123 | 1 | 5 | 112178934 | 112179086 | APC | 61 |
| 104 | AMPL393735676 | 2 | 5 | 112179087 | 112179249 | APC | 58 |
| 105 | AMPL394036510 | 1 | 5 | 112179250 | 112179419 | APC | 69 |
| 106 | AMPL394077052 | 2 | 5 | 112179420 | 112179580 | APC | 72 |
| 107 | AMPL394138546 | 1 | 5 | 112179581 | 112179758 | APC | 85 |
| 108 | AMPL567681686 | 2 | 5 | 112179759 | 112179925 | APC | 54 |
| 109 | AMPL569771076 | 1 | 6 | 31973363 | 31973522 | CYP21A2 | 107 |
| 110 | AMPL3662263761 | 2 | 6 | 31973472 | 31973603 | CYP21A2 | 94 |
| 111 | AMPL3677121820 | 1 | 6 | 31973568 | 31973698 | CYP21A2 | 81 |
| 112 | AMPL868037386 | 2 | 6 | 31973796 | 31973978 | CYP21A2 | 89 |
| 113 | AMPL868226127 | 1 | 6 | 31974024 | 31974188 | CYP21A2 | 97 |
| 114 | AMPL570682566 | 2 | 6 | 31974187 | 31974369 | CYP21A2 | 122 |
| 115 | AMPL3662870798 | 1 | 6 | 31974385 | 31974561 | CYP21A2 | 107 |
| 116 | AMPL569741665 | 2 | 6 | 31974573 | 31974718 | CYP21A2 | 75 |
| 117 | AMPL570562416 | 1 | 6 | 31974711 | 31974881 | CYP21A2 | 105 |
| 118 | AMPL569800689 | 2 | 6 | 31974961 | 31975135 | CYP21A2 | 110 |
| 119 | AMPL569809993 | 1 | 6 | 31975097 | 31975252 | CYP21A2 | 96 |
| 120 | AMPL1406102152 | 2 | 6 | 31975281 | 31975462 | CYP21A2 | 122 |
| 121 | AMPL570836129 | 1 | 6 | 31975464 | 31975638 | CYP21A2 | 119 |
| 122 | AMPL3662802661 | 2 | 6 | 31976009 | 31976168 | CYP21A2 | 112 |
| 123 | AMPL2738763272 | 1 | 6 | 31976181 | 31976269 | CYP21A2 | 54 |
| 124 | AMPL569874802 | 2 | 6 | 32006098 | 32006262 | CYP21A2 | 110 |
| 125 | AMPL868122714 | 1 | 6 | 32006523 | 32006705 | CYP21A2 | 84 |
| 126 | AMPL712390951 | 2 | 6 | 32006755 | 32006936 | CYP21A2 | 113 |
| 127 | AMPL570979062 | 1 | 6 | 32006933 | 32007107 | CYP21A2 | 118 |
| 128 | AMPL569747500 | 2 | 6 | 32007117 | 32007287 | CYP21A2 | 104 |
| 129 | AMPL3027213778 | 1 | 6 | 32007225 | 32007348 | CYP21A2 | 70 |
| 130 | AMPL3662402949 | 2 | 6 | 32007349 | 32007429 | CYP21A2 | 39 |
| 131 | AMPL3662401101 | 1 | 6 | 32007443 | 32007591 | CYP21A2 | 90 |
| 132 | AMPL712345678 | 2 | 6 | 32007585 | 32007668 | CYP21A2 | 56 |
| 133 | AMPL569737614 | 1 | 6 | 32007696 | 32007874 | CYP21A2 | 112 |
| 134 | AMPL569743668 | 2 | 6 | 32007826 | 32007983 | CYP21A2 | 98 |
| 135 | AMPL569743823 | 1 | 6 | 32007904 | 32007994 | CYP21A2 | 55 |
| 136 | AMPL868179279 | 2 | 6 | 32008022 | 32008197 | CYP21A2 | 119 |
| 137 | AMPL570761387 | 1 | 6 | 32008210 | 32008371 | CYP21A2 | 113 |
| 138 | AMPL3662395556 | 2 | 6 | 32008430 | 32008573 | CYP21A2 | 93 |
| 139 | AMPL570347419 | 1 | 6 | 32008582 | 32008743 | CYP21A2 | 116 |
| 140 | AMPL3662262933 | 2 | 6 | 32008744 | 32008902 | CYP21A2 | 113 |
| 141 | AMPL570348839 | 1 | 6 | 32008914 | 32009004 | CYP21A2 | 56 |
| 142 | AMPL561998976 | 2 | 6 | 33286461 | 33286635 | DAXX | 87 |
| 143 | AMPL561955670 | 1 | 6 | 33286701 | 33286882 | DAXX | 104 |
| 144 | AMPL3679436156 | 2 | 6 | 33286882 | 33287010 | DAXX | 67 |
| 145 | AMPL562336638 | 1 | 6 | 33287084 | 33287260 | DAXX | 92 |
| 146 | AMPL3679513190 | 2 | 6 | 33287261 | 33287414 | DAXX | 80 |
| 147 | AMPL3679558698 | 1 | 6 | 33287412 | 33287591 | DAXX | 94 |
| 148 | AMPL562160433 | 2 | 6 | 33287592 | 33287678 | DAXX | 43 |
| 149 | AMPL3679416865 | 1 | 6 | 33287766 | 33287943 | DAXX | 94 |
| 150 | AMPL3679484379 | 2 | 6 | 33287944 | 33288039 | DAXX | 54 |

FIGURE 6 (TABLE 2) (continuation)

| | | | | | | | |
|---|---|---|---|---|---|---|---|
| 151 | AMPL3679376510 | 1 | 6 | 33288138 | 33288271 | DAXX | 74 |
| 152 | AMPL3679455670 | 2 | 6 | 33288269 | 33288373 | DAXX | 60 |
| 153 | AMPL561958042 | 1 | 6 | 33288477 | 33288590 | DAXX | 62 |
| 154 | AMPL562012068 | 2 | 6 | 33288590 | 33288748 | DAXX | 98 |
| 155 | AMPL3679510308 | 1 | 6 | 33288743 | 33288914 | DAXX | 100 |
| 156 | AMPL562087991 | 2 | 6 | 33288915 | 33289040 | DAXX | 75 |
| 157 | AMPL3679568356 | 1 | 6 | 33289041 | 33289169 | DAXX | 73 |
| 158 | AMPL3679582538 | 2 | 6 | 33289170 | 33289335 | DAXX | 95 |
| 159 | AMPL1395737427 | 1 | 6 | 33289336 | 33289484 | DAXX | 82 |
| 160 | AMPL562006389 | 2 | 6 | 33289489 | 33289669 | DAXX | 107 |
| 161 | AMPL562070793 | 1 | 6 | 33289670 | 33289808 | DAXX | 81 |
| 162 | AMPL3679400237 | 2 | 6 | 33290588 | 33290743 | DAXX | 106 |
| 163 | AMPL3865810105 | 1 | 8 | 143545709 | 143545808 | BAI1 | 69 |
| 164 | AMPL3865818167 | 2 | 8 | 143545809 | 143545912 | BAI1 | 73 |
| 165 | AMPL3865821686 | 1 | 8 | 143545913 | 143546073 | BAI1 | 121 |
| 166 | AMPL3865822257 | 2 | 8 | 143546069 | 143546253 | BAI1 | 140 |
| 167 | AMPL3865825855 | 1 | 8 | 143546171 | 143546358 | BAI1 | 140 |
| 168 | AMPL800135949 | 2 | 8 | 143556774 | 143556959 | BAI1 | 141 |
| 169 | AMPL3865808206 | 1 | 8 | 143557934 | 143558111 | BAI1 | 124 |
| 170 | AMPL3865806949 | 2 | 8 | 143558462 | 143558636 | BAI1 | 123 |
| 171 | AMPL798903651 | 1 | 8 | 143558624 | 143558804 | BAI1 | 131 |
| 172 | AMPL3865826944 | 2 | 8 | 143558799 | 143558915 | BAI1 | 76 |
| 173 | AMPL3865825117 | 1 | 8 | 143559530 | 143559687 | BAI1 | 109 |
| 174 | AMPL3865831052 | 2 | 8 | 143559657 | 143559757 | BAI1 | 74 |
| 175 | AMPL798987461 | 1 | 8 | 143560626 | 143560809 | BAI1 | 130 |
| 176 | AMPL798990883 | 2 | 8 | 143560785 | 143560959 | BAI1 | 124 |
| 177 | AMPL3865808383 | 1 | 8 | 143561018 | 143561208 | BAI1 | 123 |
| 178 | AMPL3865810359 | 2 | 8 | 143562596 | 143562771 | BAI1 | 107 |
| 179 | AMPL804498886 | 1 | 8 | 143562848 | 143563018 | BAI1 | 111 |
| 180 | AMPL804512802 | 2 | 8 | 143563019 | 143563189 | BAI1 | 105 |
| 181 | AMPL3865827959 | 1 | 8 | 143565263 | 143565433 | BAI1 | 98 |
| 182 | AMPL800374648 | 2 | 8 | 143566073 | 143566251 | BAI1 | 111 |
| 183 | AMPL3865904270 | 1 | 8 | 143569700 | 143569883 | BAI1 | 118 |
| 184 | AMPL3865807813 | 2 | 8 | 143570306 | 143570398 | BAI1 | 63 |
| 185 | AMPL802990935 | 1 | 8 | 143570392 | 143570564 | BAI1 | 119 |
| 186 | AMPL803149002 | 2 | 8 | 143570591 | 143570715 | BAI1 | 89 |
| 187 | AMPL803155347 | 1 | 8 | 143570716 | 143570818 | BAI1 | 59 |
| 188 | AMPL3865922364 | 2 | 8 | 143572072 | 143572254 | BAI1 | 120 |
| 189 | AMPL799792667 | 1 | 8 | 143592271 | 143592410 | BAI1 | 109 |
| 190 | AMPL3865915576 | 2 | 8 | 143599482 | 143599647 | BAI1 | 105 |
| 191 | AMPL3865810454 | 1 | 8 | 143602140 | 143602317 | BAI1 | 103 |
| 192 | AMPL804398027 | 2 | 8 | 143603220 | 143603408 | BAI1 | 131 |
| 193 | AMPL3865811051 | 1 | 8 | 143603382 | 143603495 | BAI1 | 81 |
| 194 | AMPL3865807535 | 2 | 8 | 143603937 | 143604117 | BAI1 | 113 |
| 195 | AMPL804473858 | 1 | 8 | 143605588 | 143605770 | BAI1 | 129 |
| 196 | AMPL799107746 | 2 | 8 | 143607752 | 143607920 | BAI1 | 116 |
| 197 | AMPL799122082 | 1 | 8 | 143607921 | 143608078 | BAI1 | 102 |
| 198 | AMPL3865807845 | 2 | 8 | 143614651 | 143614749 | BAI1 | 73 |
| 199 | AMPL3865810997 | 1 | 8 | 143614722 | 143614821 | BAI1 | 63 |
| 200 | AMPL800369709 | 2 | 8 | 143618289 | 143618473 | BAI1 | 129 |
| 201 | AMPL802655922 | 1 | 8 | 143620672 | 143620858 | BAI1 | 126 |
| 202 | AMPL804499443 | 2 | 8 | 143623258 | 143623372 | BAI1 | 81 |
| 203 | AMPL3865817599 | 1 | 8 | 143623369 | 143623555 | BAI1 | 124 |
| 204 | AMPL3865827248 | 2 | 8 | 143623556 | 143623726 | BAI1 | 111 |
| 205 | AMPL804509181 | 1 | 8 | 143623989 | 143624163 | BAI1 | 128 |
| 206 | AMPL3865811390 | 2 | 8 | 143624686 | 143624829 | BAI1 | 88 |
| 207 | AMPL3865824181 | 1 | 8 | 143624944 | 143625106 | BAI1 | 109 |
| 208 | AMPL803999478 | 2 | 8 | 143625532 | 143625695 | BAI1 | 117 |
| 209 | AMPL3865823396 | 1 | 8 | 143625665 | 143625754 | BAI1 | 65 |
| 210 | AMPL3665051746 | 1 | 9 | 21968182 | 21968339 | CDKN2A | 81 |
| 211 | AMPL1184678398 | 2 | 9 | 21968642 | 21968826 | CDKN2A | 99 |
| 212 | AMPL391621543 | 1 | 9 | 21970895 | 21971073 | CDKN2A | 130 |
| 213 | AMPL703866942 | 2 | 9 | 21971049 | 21971219 | CDKN2A | 125 |
| 214 | AMPL403321696 | 1 | 9 | 21974450 | 21974612 | CDKN2A | 93 |
| 215 | AMPL395029367 | 2 | 9 | 21974612 | 21974792 | CDKN2A | 129 |
| 216 | AMPL1821497782 | 1 | 9 | 21974790 | 21974968 | CDKN2A | 133 |
| 217 | AMPL1185278949 | 2 | 9 | 21994132 | 21994316 | CDKN2A | 128 |
| 218 | AMPL1185303069 | 1 | 9 | 21994317 | 21994413 | CDKN2A | 70 |
| 219 | AMPL909388873 | 1 | 10 | 104590379 | 104590517 | CYP17A1 | 80 |
| 220 | AMPL909420642 | 2 | 10 | 104590518 | 104590673 | CYP17A1 | 93 |
| 221 | AMPL909474231 | 1 | 10 | 104590673 | 104590843 | CYP17A1 | 105 |
| 222 | AMPL909108041 | 2 | 10 | 104591149 | 104591289 | CYP17A1 | 85 |
| 223 | AMPL4042737750 | 1 | 10 | 104591289 | 104591402 | CYP17A1 | 60 |
| 224 | AMPL909107601 | 2 | 10 | 104592235 | 104592372 | CYP17A1 | 88 |

FIGURE 6 (TABLE 2) (continuation)

| | | | | | | | |
|---|---|---|---|---|---|---|---|
| 225 | AMPL909134907 | 1 | 10 | 104592373 | 104592454 | CYP17A1 | 41 |
| 226 | AMPL909072886 | 2 | 10 | 104592667 | 104592832 | CYP17A1 | 96 |
| 227 | AMPL909145618 | 1 | 10 | 104592833 | 104592977 | CYP17A1 | 71 |
| 228 | AMPL908319985 | 2 | 10 | 104593784 | 104593902 | CYP17A1 | 36 |
| 229 | AMPL4042719006 | 1 | 10 | 104594524 | 104594644 | CYP17A1 | 61 |
| 230 | AMPL4042783864 | 2 | 10 | 104594645 | 104594788 | CYP17A1 | 71 |
| 231 | AMPL908889303 | 1 | 10 | 104594846 | 104595023 | CYP17A1 | 109 |
| 232 | AMPL908962869 | 2 | 10 | 104595023 | 104595168 | CYP17A1 | 84 |
| 233 | AMPL4042699352 | 1 | 10 | 104596778 | 104596900 | CYP17A1 | 73 |
| 234 | AMPL4042733400 | 2 | 10 | 104596901 | 104597073 | CYP17A1 | 95 |
| 235 | AMPL908549044 | 1 | 10 | 104597074 | 104597155 | CYP17A1 | 47 |
| 236 | AMPL415118570 | 1 | 11 | 64571797 | 64571913 | MEN1 | 63 |
| 237 | AMPL415139377 | 2 | 11 | 64571914 | 64572101 | MEN1 | 121 |
| 238 | AMPL415146260 | 1 | 11 | 64572092 | 64572265 | MEN1 | 134 |
| 239 | AMPL415147286 | 2 | 11 | 64572151 | 64572304 | MEN1 | 118 |
| 240 | AMPL420236215 | 1 | 11 | 64572449 | 64572604 | MEN1 | 98 |
| 241 | AMPL420288269 | 2 | 11 | 64572602 | 64572693 | MEN1 | 62 |
| 242 | AMPL415709890 | 1 | 11 | 64573022 | 64573197 | MEN1 | 114 |
| 243 | AMPL437960938 | 2 | 11 | 64573198 | 64573354 | MEN1 | 99 |
| 244 | AMPL413367150 | 1 | 11 | 64573656 | 64573793 | MEN1 | 87 |
| 245 | AMPL413382245 | 2 | 11 | 64573794 | 64573937 | MEN1 | 90 |
| 246 | AMPL414658635 | 1 | 11 | 64574453 | 64574633 | MEN1 | 116 |
| 247 | AMPL414187227 | 2 | 11 | 64574592 | 64574746 | MEN1 | 97 |
| 248 | AMPL413952293 | 1 | 11 | 64575051 | 64575171 | MEN1 | 67 |
| 249 | AMPL414551647 | 2 | 11 | 64575299 | 64575456 | MEN1 | 100 |
| 250 | AMPL414568938 | 1 | 11 | 64575447 | 64575584 | MEN1 | 88 |
| 251 | AMPL416422568 | 2 | 11 | 64577107 | 64577259 | MEN1 | 86 |
| 252 | AMPL416509330 | 1 | 11 | 64577259 | 64577434 | MEN1 | 119 |
| 253 | AMPL416538242 | 2 | 11 | 64577434 | 64577546 | MEN1 | 73 |
| 254 | AMPL416539471 | 1 | 11 | 64577540 | 64577670 | MEN1 | 101 |
| 255 | AMPL3653606322 | 2 | 11 | 119180525 | 119180703 | MCAM | 98 |
| 256 | AMPL3653632931 | 1 | 11 | 119180988 | 119181125 | MCAM | 89 |
| 257 | AMPL3653645532 | 2 | 11 | 119181126 | 119181237 | MCAM | 64 |
| 258 | AMPL3653689329 | 1 | 11 | 119181435 | 119181619 | MCAM | 120 |
| 259 | AMPL3653589885 | 2 | 11 | 119181736 | 119181916 | MCAM | 107 |
| 260 | AMPL1683718633 | 1 | 11 | 119181937 | 119182093 | MCAM | 100 |
| 261 | AMPL1684089945 | 2 | 11 | 119182091 | 119182246 | MCAM | 94 |
| 262 | AMPL3653609678 | 1 | 11 | 119182218 | 119182389 | MCAM | 102 |
| 263 | AMPL3653625890 | 2 | 11 | 119182455 | 119182547 | MCAM | 52 |
| 264 | AMPL3865866652 | 1 | 11 | 119182536 | 119182667 | MCAM | 85 |
| 265 | AMPL3653642615 | 2 | 11 | 119182741 | 119182897 | MCAM | 98 |
| 266 | AMPL3653651392 | 1 | 11 | 119182876 | 119182995 | MCAM | 75 |
| 267 | AMPL3653655738 | 2 | 11 | 119182996 | 119183147 | MCAM | 91 |
| 268 | AMPL3653630720 | 1 | 11 | 119183205 | 119183390 | MCAM | 110 |
| 269 | AMPL3653653453 | 2 | 11 | 119183415 | 119183556 | MCAM | 81 |
| 270 | AMPL3653662160 | 1 | 11 | 119183557 | 119183733 | MCAM | 101 |
| 271 | AMPL3865881680 | 2 | 11 | 119185187 | 119185369 | MCAM | 111 |
| 272 | AMPL3653577615 | 1 | 11 | 119185334 | 119185513 | MCAM | 108 |
| 273 | AMPL3653587798 | 2 | 11 | 119185466 | 119185625 | MCAM | 99 |
| 274 | AMPL3865869828 | 1 | 11 | 119185621 | 119185761 | MCAM | 91 |
| 275 | AMPL3865880050 | 2 | 11 | 119185798 | 119185887 | MCAM | 56 |
| 276 | AMPL3653656647 | 1 | 11 | 119185873 | 119186052 | MCAM | 128 |
| 277 | AMPL3653646407 | 2 | 11 | 119187653 | 119187837 | MCAM | 140 |
| 278 | AMPL405668467 | 1 | 13 | 48878023 | 48878134 | RB1 | 89 |
| 279 | AMPL405668791 | 2 | 13 | 48878062 | 48878182 | RB1 | 95 |
| 280 | AMPL425078137 | 1 | 13 | 48878183 | 48878351 | RB1 | 133 |
| 281 | AMPL1687195295 | 2 | 13 | 48881338 | 48881469 | RB1 | 36 |
| 282 | AMPL408897671 | 1 | 13 | 48881447 | 48881526 | RB1 | 29 |
| 283 | AMPL3499037056 | 2 | 13 | 48916600 | 48916753 | RB1 | 37 |
| 284 | AMPL3677824944 | 1 | 13 | 48916754 | 48916898 | RB1 | 52 |
| 285 | AMPL782966304 | 2 | 13 | 48919131 | 48919298 | RB1 | 45 |
| 286 | AMPL3677839183 | 1 | 13 | 48919299 | 48919378 | RB1 | 23 |
| 287 | AMPL426521876 | 2 | 13 | 48921810 | 48921974 | RB1 | 35 |
| 288 | AMPL410559974 | 1 | 13 | 48921970 | 48922078 | RB1 | 30 |
| 289 | AMPL449040401 | 2 | 13 | 48922945 | 48923109 | RB1 | 43 |
| 290 | AMPL417128043 | 1 | 13 | 48923126 | 48923275 | RB1 | 41 |
| 291 | AMPL1078580037 | 2 | 13 | 48934110 | 48934242 | RB1 | 42 |
| 292 | AMPL418295944 | 1 | 13 | 48936831 | 48937002 | RB1 | 54 |
| 293 | AMPL1074269905 | 2 | 13 | 48936991 | 48937144 | RB1 | 49 |
| 294 | AMPL411036386 | 1 | 13 | 48939103 | 48939210 | RB1 | 31 |
| 295 | AMPL416346900 | 2 | 13 | 48941593 | 48941759 | RB1 | 47 |
| 296 | AMPL418757130 | 1 | 13 | 48942527 | 48942702 | RB1 | 49 |
| 297 | AMPL651427926 | 2 | 13 | 48942703 | 48942793 | RB1 | 27 |
| 298 | AMPL1076140421 | 1 | 13 | 48947525 | 48947654 | RB1 | 38 |
| 299 | AMPL410025321 | 2 | 13 | 48951047 | 48951212 | RB1 | 59 |
| 300 | AMPL1558219837 | 1 | 13 | 48954321 | 48954456 | RB1 | 47 |
| 301 | AMPL782507847 | 2 | 13 | 48955309 | 48955477 | RB1 | 48 |

FIGURE 6 (TABLE 2) (continuation)

| | | | | | | | |
|---|---|---|---|---|---|---|---|
| 302 | AMPL409852039 | 1 | 13 | 48955453 | 48955599 | RB1 | 50 |
| 303 | AMPL408333150 | 2 | 13 | 49027030 | 49027188 | RB1 | 49 |
| 304 | AMPL637245808 | 1 | 13 | 49027189 | 49027266 | RB1 | 28 |
| 305 | AMPL2452020914 | 2 | 13 | 49030279 | 49030447 | RB1 | 65 |
| 306 | AMPL448901123 | 1 | 13 | 49030457 | 49030629 | RB1 | 61 |
| 307 | AMPL2520015974 | 2 | 13 | 49033708 | 49033838 | RB1 | 38 |
| 308 | AMPL2520017411 | 1 | 13 | 49033839 | 49033974 | RB1 | 62 |
| 309 | AMPL417468440 | 2 | 13 | 49037846 | 49037954 | RB1 | 32 |
| 310 | AMPL408736586 | 1 | 13 | 49037909 | 49038012 | RB1 | 33 |
| 311 | AMPL414695272 | 2 | 13 | 49039020 | 49039183 | RB1 | 34 |
| 312 | AMPL651771571 | 1 | 13 | 49039154 | 49039274 | RB1 | 44 |
| 313 | AMPL406167024 | 2 | 13 | 49039348 | 49039519 | RB1 | 74 |
| 314 | AMPL405360749 | 1 | 13 | 49047458 | 49047601 | RB1 | 36 |
| 315 | AMPL912027424 | 2 | 13 | 49050717 | 49050887 | RB1 | 55 |
| 316 | AMPL408928607 | 1 | 13 | 49050888 | 49050984 | RB1 | 42 |
| 317 | AMPL410859997 | 2 | 13 | 49051421 | 49051580 | RB1 | 49 |
| 318 | AMPL407755528 | 1 | 13 | 49054081 | 49054248 | RB1 | 72 |
| 319 | AMPL388592559 | 1 | 17 | 7572848 | 7573025 | TP53 | 93 |
| 320 | AMPL387894777 | 2 | 17 | 7573864 | 7574041 | TP53 | 110 |
| 321 | AMPL3658613517 | 1 | 17 | 7576617 | 7576686 | TP53 | 20 |
| 322 | AMPL1514447650 | 2 | 17 | 7576790 | 7576968 | TP53 | 82 |
| 323 | AMPL705603720 | 1 | 17 | 7576996 | 7577174 | TP53 | 101 |
| 324 | AMPL388733576 | 2 | 17 | 7577357 | 7577509 | TP53 | 93 |
| 325 | AMPL387805648 | 1 | 17 | 7577509 | 7577613 | TP53 | 57 |
| 326 | AMPL387845397 | 2 | 17 | 7578151 | 7578333 | TP53 | 99 |
| 327 | AMPL2449945349 | 1 | 17 | 7578250 | 7578425 | TP53 | 106 |
| 328 | AMPL707393441 | 2 | 17 | 7578426 | 7578560 | TP53 | 83 |
| 329 | AMPL624460718 | 1 | 17 | 7579235 | 7579385 | TP53 | 82 |
| 330 | AMPL651471237 | 2 | 17 | 7579386 | 7579522 | TP53 | 91 |
| 331 | AMPL388679445 | 1 | 17 | 7579523 | 7579684 | TP53 | 99 |
| 332 | AMPL1547505015 | 2 | 17 | 7579610 | 7579766 | TP53 | 92 |
| 333 | AMPL388009515 | 1 | 17 | 7579783 | 7579964 | TP53 | 110 |
| 334 | AMPL899064867 | 2 | 17 | 56432242 | 56432419 | RNF43 | 102 |
| 335 | AMPL3709462843 | 1 | 17 | 56434806 | 56434974 | RNF43 | 105 |
| 336 | AMPL3709475101 | 2 | 17 | 56434975 | 56435083 | RNF43 | 65 |
| 337 | AMPL899038182 | 1 | 17 | 56435080 | 56435214 | RNF43 | 84 |
| 338 | AMPL899045005 | 2 | 17 | 56435215 | 56435351 | RNF43 | 88 |
| 339 | AMPL898992061 | 1 | 17 | 56435351 | 56435525 | RNF43 | 109 |
| 340 | AMPL899021677 | 2 | 17 | 56435508 | 56435637 | RNF43 | 78 |
| 341 | AMPL899034334 | 1 | 17 | 56435638 | 56435790 | RNF43 | 89 |
| 342 | AMPL898991403 | 2 | 17 | 56435791 | 56435893 | RNF43 | 68 |
| 343 | AMPL898997152 | 1 | 17 | 56435880 | 56436059 | RNF43 | 126 |
| 344 | AMPL899002910 | 2 | 17 | 56436056 | 56436215 | RNF43 | 88 |
| 345 | AMPL899008783 | 1 | 17 | 56437472 | 56437645 | RNF43 | 96 |
| 346 | AMPL3709460252 | 2 | 17 | 56438131 | 56438316 | RNF43 | 120 |
| 347 | AMPL899028869 | 1 | 17 | 56439851 | 56440028 | RNF43 | 110 |
| 348 | AMPL3344091797 | 2 | 17 | 56440594 | 56440678 | RNF43 | 51 |
| 349 | AMPL3344040552 | 1 | 17 | 56440679 | 56440824 | RNF43 | 86 |
| 350 | AMPL898989797 | 2 | 17 | 56440818 | 56441000 | RNF43 | 109 |
| 351 | AMPL899064539 | 1 | 17 | 56448254 | 56448412 | RNF43 | 95 |
| 352 | AMPL3343964183 | 2 | 17 | 56492569 | 56492733 | RNF43 | 57 |
| 353 | AMPL3343976985 | 1 | 17 | 56492734 | 56492836 | RNF43 | 47 |
| 354 | AMPL3343980870 | 2 | 17 | 56492834 | 56492950 | RNF43 | 77 |
| 355 | AMPL3653639719 | 1 | 22 | 29279938 | 29280091 | ZNRF3 | 115 |
| 356 | AMPL3653601397 | 2 | 22 | 29383038 | 29383206 | ZNRF3 | 80 |
| 357 | AMPL3865922136 | 1 | 22 | 29438452 | 29438606 | ZNRF3 | 82 |
| 358 | AMPL3653718993 | 2 | 22 | 29439234 | 29439345 | ZNRF3 | 59 |
| 359 | AMPL3653724262 | 1 | 22 | 29439346 | 29439453 | ZNRF3 | 63 |
| 360 | AMPL3653596623 | 2 | 22 | 29440727 | 29440915 | ZNRF3 | 101 |
| 361 | AMPL3653664878 | 1 | 22 | 29442545 | 29442724 | ZNRF3 | 93 |
| 362 | AMPL3653695737 | 2 | 22 | 29442725 | 29442902 | ZNRF3 | 105 |
| 363 | AMPL3653606591 | 1 | 22 | 29444332 | 29444513 | ZNRF3 | 111 |
| 364 | AMPL3653597794 | 2 | 22 | 29445143 | 29445320 | ZNRF3 | 109 |
| 365 | AMPL3653611840 | 1 | 22 | 29445320 | 29445410 | ZNRF3 | 61 |
| 366 | AMPL3653615770 | 2 | 22 | 29445404 | 29445579 | ZNRF3 | 118 |
| 367 | AMPL3653620941 | 1 | 22 | 29445579 | 29445758 | ZNRF3 | 128 |
| 368 | AMPL3653625221 | 2 | 22 | 29445758 | 29445873 | ZNRF3 | 77 |
| 369 | AMPL3653627935 | 1 | 22 | 29445873 | 29445956 | ZNRF3 | 53 |
| 370 | AMPL3653628605 | 2 | 22 | 29445954 | 29446135 | ZNRF3 | 138 |
| 371 | AMPL3653631627 | 1 | 22 | 29446094 | 29446217 | ZNRF3 | 83 |
| 372 | AMPL3653574252 | 2 | 22 | 29446218 | 29446386 | ZNRF3 | 121 |
| 373 | AMPL3653577531 | 1 | 22 | 29446373 | 29446514 | ZNRF3 | 93 |
| 374 | AMPL3653582249 | 2 | 22 | 29446515 | 29446698 | ZNRF3 | 124 |
| 375 | AMPL3653590197 | 1 | 22 | 29446689 | 29446867 | ZNRF3 | 133 |
| 376 | AMPL3865911109 | 2 | 22 | 29446826 | 29446947 | ZNRF3 | 82 |
| 377 | AMPL3653700400 | 1 | 22 | 29449467 | 29449642 | ZNRF3 | 106 |
| 378 | AMPL3711836310 | 1 | 23 | 70338561 | 70338714 | MED12 | 103 |

FIGURE 6 (TABLE 2) (continuation)

| | | | | | | | |
|---|---|---|---|---|---|---|---|
| 379 | AMPL651714093 | 2 | 23 | 70339215 | 70339347 | MED12 | 69 |
| 380 | AMPL906795268 | 1 | 23 | 70339408 | 70339569 | MED12 | 79 |
| 381 | AMPL3711866674 | 2 | 23 | 70339570 | 70339733 | MED12 | 83 |
| 382 | AMPL3009489352 | 1 | 23 | 70339695 | 70339873 | MED12 | 89 |
| 383 | AMPL3709434343 | 2 | 23 | 70339874 | 70340025 | MED12 | 73 |
| 384 | AMPL906106931 | 1 | 23 | 70340779 | 70340857 | MED12 | 34 |
| 385 | AMPL3006693692 | 2 | 23 | 70340858 | 70341030 | MED12 | 99 |
| 386 | AMPL3711844614 | 1 | 23 | 70341158 | 70341301 | MED12 | 75 |
| 387 | AMPL2877638682 | 2 | 23 | 70341348 | 70341513 | MED12 | 89 |
| 388 | AMPL2877514477 | 1 | 23 | 70341514 | 70341688 | MED12 | 100 |
| 389 | AMPL3709391663 | 2 | 23 | 70342028 | 70342206 | MED12 | 93 |
| 390 | AMPL909516142 | 1 | 23 | 70342321 | 70342494 | MED12 | 82 |
| 391 | AMPL3004799112 | 2 | 23 | 70342425 | 70342594 | MED12 | 78 |
| 392 | AMPL3013557912 | 1 | 23 | 70342595 | 70342765 | MED12 | 78 |
| 393 | AMPL907059804 | 2 | 23 | 70342917 | 70343087 | MED12 | 91 |
| 394 | AMPL906100900 | 1 | 23 | 70343420 | 70343591 | MED12 | 92 |
| 395 | AMPL904998147 | 2 | 23 | 70343896 | 70344074 | MED12 | 79 |
| 396 | AMPL905037727 | 1 | 23 | 70344075 | 70344245 | MED12 | 97 |
| 397 | AMPL906277894 | 2 | 23 | 70344562 | 70344739 | MED12 | 87 |
| 398 | AMPL906316334 | 1 | 23 | 70344662 | 70344838 | MED12 | 81 |
| 399 | AMPL907606591 | 2 | 23 | 70344839 | 70345011 | MED12 | 101 |
| 400 | AMPL907675513 | 1 | 23 | 70345183 | 70345352 | MED12 | 92 |
| 401 | AMPL905739431 | 2 | 23 | 70345450 | 70345623 | MED12 | 95 |
| 402 | AMPL907563466 | 1 | 23 | 70345845 | 70346026 | MED12 | 105 |
| 403 | AMPL906192477 | 2 | 23 | 70346170 | 70346351 | MED12 | 97 |
| 404 | AMPL3017809238 | 1 | 23 | 70346675 | 70346825 | MED12 | 86 |
| 405 | AMPL3709402937 | 2 | 23 | 70346826 | 70346996 | MED12 | 94 |
| 406 | AMPL905547750 | 1 | 23 | 70347154 | 70347340 | MED12 | 97 |
| 407 | AMPL908974353 | 2 | 23 | 70347658 | 70347837 | MED12 | 90 |
| 408 | AMPL3711875843 | 1 | 23 | 70347838 | 70347977 | MED12 | 76 |
| 409 | AMPL3011141139 | 2 | 23 | 70348020 | 70348190 | MED12 | 85 |
| 410 | AMPL3006620377 | 1 | 23 | 70348191 | 70348361 | MED12 | 92 |
| 411 | AMPL907670382 | 2 | 23 | 70348426 | 70348601 | MED12 | 85 |
| 412 | AMPL909628174 | 1 | 23 | 70348924 | 70349090 | MED12 | 85 |
| 413 | AMPL907244830 | 2 | 23 | 70349134 | 70349312 | MED12 | 104 |
| 414 | AMPL3006524278 | 1 | 23 | 70349439 | 70349563 | MED12 | 73 |
| 415 | AMPL3709432211 | 2 | 23 | 70349564 | 70349716 | MED12 | 89 |
| 416 | AMPL3709391687 | 1 | 23 | 70349840 | 70349991 | MED12 | 84 |
| 417 | AMPL905965443 | 2 | 23 | 70349992 | 70350105 | MED12 | 70 |
| 418 | AMPL3709402114 | 1 | 23 | 70351330 | 70351508 | MED12 | 100 |
| 419 | AMPL905538010 | 2 | 23 | 70351887 | 70352064 | MED12 | 92 |
| 420 | AMPL3711824167 | 1 | 23 | 70352209 | 70352395 | MED12 | 101 |
| 421 | AMPL908588251 | 2 | 23 | 70352566 | 70352734 | MED12 | 80 |
| 422 | AMPL908718348 | 1 | 23 | 70352735 | 70352847 | MED12 | 66 |
| 423 | AMPL905540967 | 2 | 23 | 70352923 | 70353106 | MED12 | 85 |
| 424 | AMPL905507552 | 1 | 23 | 70354166 | 70354343 | MED12 | 108 |
| 425 | AMPL906862728 | 2 | 23 | 70354546 | 70354718 | MED12 | 91 |
| 426 | AMPL3709403782 | 1 | 23 | 70354914 | 70355069 | MED12 | 90 |
| 427 | AMPL1442180178 | 2 | 23 | 70355070 | 70355153 | MED12 | 43 |
| 428 | AMPL906616858 | 1 | 23 | 70356037 | 70356214 | MED12 | 83 |
| 429 | AMPL906697834 | 2 | 23 | 70356210 | 70356379 | MED12 | 112 |
| 430 | AMPL906724175 | 1 | 23 | 70356380 | 70356533 | MED12 | 91 |
| 431 | AMPL1615271560 | 2 | 23 | 70356696 | 70356815 | MED12 | 73 |
| 432 | AMPL3711756658 | 1 | 23 | 70356816 | 70356897 | MED12 | 46 |
| 433 | AMPL908318402 | 2 | 23 | 70356983 | 70357162 | MED12 | 104 |
| 434 | AMPL908387874 | 1 | 23 | 70357163 | 70357315 | MED12 | 93 |
| 435 | AMPL906131345 | 2 | 23 | 70357357 | 70357534 | MED12 | 92 |
| 436 | AMPL906991969 | 1 | 23 | 70357513 | 70357688 | MED12 | 101 |
| 437 | AMPL907057590 | 2 | 23 | 70357689 | 70357799 | MED12 | 65 |
| 438 | AMPL3711831192 | 1 | 23 | 70360461 | 70360564 | MED12 | 57 |
| 439 | AMPL1657944959 | 2 | 23 | 70360543 | 70360713 | MED12 | 107 |
| 440 | AMPL794433216 | 1 | 23 | 70361056 | 70361242 | MED12 | 109 |
| 441 | AMPL3711817288 | 2 | 23 | 70361686 | 70361854 | MED12 | 90 |
| 442 | AMPL932145015 | 1 | 23 | 70361962 | 70362143 | MED12 | 98 |

FIGURE 6 (TABLE 2) (end)

| | Sample | Gene | CN | MBAF | Call | Call_distance | Proportion_of_cells | N_amplicons | N_snpHet | Chr | Start_37 | End_37 | Start_Ind | End_Ind |
|---|---|---|---|---|---|---|---|---|---|---|---|---|---|---|
| 1 | ACC100 | HSD3B2 | 0,53 | NA | 1 DNA copy? | NA | NA | 9 | 0 | 1 | 119958027 | 119965088 | 1 | 9 |
| 2 | ACC100 | CTNNB1 | 0,51 | NA | 1 DNA copy? | NA | NA | 25 | 0 | 3 | 41265452 | 41280734 | 10 | 34 |
| 3 | ACC100 | APC | 0,81 | 0,64 | Chromosome Loss | 0,04 | 0,29 | 73 | 5 | 5 | 112043355 | 112179759 | 35 | 107 |
| 4 | ACC100 | CYP21A2 | 0,74 | 0,92 | Chromosome Loss | 0,16 | 0,85 | 29 | 3 | 6 | 31973363 | 32008914 | 108 | 136 |
| 5 | ACC100 | DAXX | 0,66 | 0,94 | Chromosome Loss | 0,10 | 0,88 | 20 | 1 | 6 | 33286461 | 33289670 | 137 | 156 |
| 6 | ACC100 | BAI1 | 0,63 | 0,55 | Diploid_het | 0,38 | NA | 42 | 2 | 8 | 143545709 | 143625665 | 157 | 198 |
| 7 | ACC100 | CDKN2A | 0,04 | NA | Homozygous Deletion | NA | NA | 7 | NA | 9 | 21968182 | 21994413 | 199 | 205 |
| 8 | ACC100 | CYP17A1 | 0,30 | 0,90 | Chromosome Loss | -0,30 | 0,80 | 17 | 6 | 10 | 104590379 | 104597074 | 206 | 222 |
| 9 | ACC100 | MEN1 | 0,46 | 0,95 | Chromosome Loss | -0,09 | 0,91 | 18 | 1 | 11 | 64571797 | 64577434 | 223 | 240 |
| 10 | ACC100 | MCAM | 0,66 | 0,93 | Chromosome Loss | 0,09 | 0,86 | 21 | 1 | 11 | 119180525 | 119185873 | 241 | 261 |
| 11 | ACC100 | RB1 | 0,64 | NA | 1 DNA copy? | NA | NA | 40 | 0 | 13 | 48878023 | 49054081 | 262 | 301 |
| 12 | ACC100 | TP53 | 0,50 | NA | 1 DNA copy? | NA | NA | 15 | 0 | 17 | 7572848 | 7579783 | 302 | 316 |
| 13 | ACC100 | RNF43 | 0,55 | 0,95 | Chromosome Loss | 0,00 | 0,90 | 20 | 4 | 17 | 56432242 | 56492834 | 317 | 336 |
| 14 | ACC100 | ZNRF3 | 0,53 | 0,93 | Chromosome Loss | -0,04 | 0,86 | 20 | 2 | 22 | 29383038 | 29449467 | 337 | 356 |
| 15 | ACC100 | MED12 | 0,59 | 0,92 | Chromosome Loss | 0,01 | 0,84 | 65 | 5 | 23 | 70338561 | 70361962 | 357 | 421 |
| 16 | ACC101 | HSD3B2 | 0,63 | NA | 1 DNA copy? | NA | NA | 9 | 0 | 1 | 119958027 | 119965088 | 1 | 9 |
| 17 | ACC101 | CTNNB1 | 0,89 | NA | 2 DNA copies? | NA | NA | 25 | 0 | 3 | 41265452 | 41280734 | 10 | 34 |
| 18 | ACC101 | APC | 1,03 | 0,52 | Diploid_het | 0,04 | NA | 73 | 6 | 5 | 112043355 | 112179759 | 35 | 107 |
| 19 | ACC101 | CYP21A2 | 0,59 | 0,55 | Diploid_het | 0,42 | NA | 29 | 4 | 6 | 31973363 | 32008914 | 108 | 136 |
| 20 | ACC101 | DAXX | 0,82 | 0,55 | Diploid_het | 0,18 | NA | 20 | 1 | 6 | 33286461 | 33289670 | 137 | 156 |

FIGURE 7 (TABLE 6)

Europäisches Patentamt
European Patent Office
Office européen des brevets

# EUROPEAN SEARCH REPORT

Application Number
EP 17 30 5968

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHENG SIYUAN ET AL: "Comprehensive Pan-Genomic Characterization of Adrenocortical Carcinoma", CANCER CELL, CELL PRESS, US, vol. 29, no. 5, 9 May 2016 (2016-05-09), pages 723-736, XP029533970, ISSN: 1535-6108, DOI: 10.1016/J.CCELL.2016.04.002 | 1-6,8-15 | INV. C12Q1/68 G06F19/22 |
| Y | * abstract; figure 4 * * page 725, column 1 * ----- | 7-11, 13-16 | |
| X | KIRSTEN M TIMMS ET AL: "Association of BRCA1/2 defects with genomic scores predictive of DNA damage repair deficiency among breast cancer subtypes", BREAST CANCER RESEARCH, CURRENT MEDICINE GROUP LTD, GB, vol. 16, no. 6, 5 December 2014 (2014-12-05), pages 1-9, XP021210371, ISSN: 1465-5411, DOI: 10.1186/S13058-014-0475-X | 1-6,12 | |
| Y | * abstract * ----- | 7-11, 13-16 | TECHNICAL FIELDS SEARCHED (IPC) C12Q G06F |
| Y | WO 2010/075570 A2 (UNIV NEW YORK [US]; MISHRA BHUBANESWAR [US]; NARZISI GIUSEPPE [US]) 1 July 2010 (2010-07-01) | 7,16 | |
| A | * abstract; claims 3,29 * * paragraph [0006] * ----- | 1-6,8-15 | |
| A | EP 3 118 324 A1 (CARTAGENIA N V [BE]) 18 January 2017 (2017-01-18) * abstract; claim 1 * ----- | 1-16 | |
| A | WO 2004/086949 A2 (WAYNE JOHN CANCER INST [US]; HOON DAVE S B [US]; TABACK BRET [US]) 14 October 2004 (2004-10-14) * abstract; claim 1 * ----- | 1-16 | |

The present search report has been drawn up for all claims

1

| Place of search | Date of completion of the search | Examiner |
|---|---|---|
| Munich | 17 November 2017 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 30 5968

This annex lists the patent family members relating to the patent documents cited in the above-mentioned European search report. The members are as contained in the European Patent Office EDP file on
The European Patent Office is in no way liable for these particulars which are merely given for the purpose of information.

17-11-2017

| Patent document cited in search report | Publication date | Patent family member(s) | Publication date |
|---|---|---|---|
| WO 2010075570 A2 | 01-07-2010 | US 2012041727 A1<br>WO 2010075570 A2 | 16-02-2012<br>01-07-2010 |
| EP 3118324 A1 | 18-01-2017 | NONE | |
| WO 2004086949 A2 | 14-10-2004 | US 2006051768 A1<br>US 2010330567 A1<br>US 2011097727 A1<br>WO 2004086949 A2 | 09-03-2006<br>30-12-2010<br>28-04-2011<br>14-10-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZHENG S ; CHERNIACK AD ; DEWAL N ; MOFFITT RA ; DANILOVA L ; MURRAY BA ; LERARIO AM ; ELSE T ; KNIJNENBURG TA ; CIRIELLO G.** Comprehensive Pan-Genomic Characterization of Adrenocortical Carcinoma. *Cancer Cell,* 2016, vol. 29, 723-736
- **HRDLICKOVA R ; TOLOUE M ; TIAN B.** RNA-Seq methods for transcriptome analysis: RNA-Seq. *Wiley Interdisciplinary Reviews: RNA,* 2016, http://doi.wiley.com/10.1002/wrna.1364
- **HAUSSER J ; ZAVOLAN M.** Identification and consequences of miRNA-target interactions - beyond repression of gene expression. *Nature Reviews Genetics,* 2014, vol. 15, 599-612
- **RUSSO CD ; DI GIACOMO G ; CIGNINI P ; PADULA F ; MANGIAFICO L ; MESORACA A ; D'EMIDIO L ; MCCLUSKEY MR ; PAGANELLI A ; GIORLANDINO C.** Comparative study of aCGH and Next Generation Sequencing (NGS) for chromosomal microdeletion and microduplication screening. *Journal of prenatal medicine,* 2014, vol. 8, 57
- **ABEL HJ ; DUNCAVAGE EJ.** Detection of structural DNA variation from next generation sequencing data: a review of informatic approaches. *Cancer Genetics,* 2013, vol. 206, 432-440
- **ASSIÉ G ; JOUINOT A ; BERTHERAT J.** The "omics" of adrenocortical tumours for personalized medicine. *Nature Reviews Endocrinology,* 2014, vol. 10, 215-228
- **WEISENBERGER DJ.** Characterizing DNA methylation alterations from The Cancer Genome Atlas. *Journal of Clinical Investigation,* 2014, vol. 124, 17-23
- Sequencing the Cancer Methylome. **SHULL AY ; NOONEPALLE SK ; LEE E-J ; CHOI J-H ; SHI H.** Cancer Epigenetics. Springer New York, 2015, 627-651
- **SONNET M ; BAER C ; REHLI M ; WEICHENHAN D ; PLASS C.** Enrichment of methylated DNA by methyl-CpG immunoprecipitation. *Methods Mol Biol,* 2013, vol. 971, 201-212
- **OFFIT K.** Decade in review-genomics: A decade of discovery in cancer genomics. *Nature Reviews Clinical Oncology,* 2014, vol. 11, 632-634
- **CLINE MS ; CRAFT B ; SWATLOSKI T ; GOLDMAN M ; MA S ; HAUSSLER D ; ZHU J.** Exploring TCGA Pan-Cancer Data at the UCSC Cancer Genomics Browser. *Scientific Reports,* 2013, vol. 3, http://www.nature.com/articles/srep02652
- **ZHAO Q ; SHI X ; XIE Y ; HUANG J ; SHIA B ; MA S.** Combining multidimensional genomic measurements for predicting cancer prognosis: observations from TCGA. *Briefings in Bioinformatics,* 2015, vol. 16, 291-303
- **NEAPOLITAN R ; HORVATH CM ; JIANG X.** Pan-cancer analysis of TCGA data reveals notable signaling pathways. *BMC Cancer,* 2015, vol. 15, http://www.biomedcentral.com/1471-2407/15/516
- **NAKAGAWA H ; WARDELL CP ; FURUTA M ; TANIGUCHI H ; FUJIMOTO A.** Cancer whole-genome sequencing: present and future. *Oncogene,* 2015, vol. 34, 5943-5950
- **CHING T ; PEPLOWSKA K ; HUANG S ; ZHU X ; SHEN Y ; MOLNAR J ; YU H ; TIIRIKAINEN M ; FOGELGREN B ; FAN R.** Pan-Cancer Analyses Reveal Long Intergenic Non-Coding RNAs Relevant to Tumor Diagnosis, Subtyping and Prognosis. *EBioMedicine,* 2016, vol. 7, 62-72
- **VOCKLEY JG ; NIEDERHUBER JE.** Diagnosis and treatment of cancer using genomics. *BMJ,* 2015, vol. 350, h1832-h1832
- Lung Cancer Genomics in the Era of Accelerated Targeted Drug Development. **WIJESINGHE P ; BOLLIG-FISCHER A.** Lung Cancer and Personalized Medicine: Novel Therapies and Clinical Management. Springer International Publishing, 2016, 1-23
- **TANG B ; HSU P-Y ; HUANG TH-M ; JIN VX.** Cancer omics: From regulatory networks to clinical outcomes. *Cancer Letters,* 2013, vol. 340, 277-283
- **DESAI A ; JERE A.** Next-generation sequencing: ready for the clinics. *Clinical Genetics,* 2012, vol. 81, 503-510
- **XUAN J ; YU Y ; QING T ; GUO L ; SHI L.** Next-generation sequencing in the clinic: Promises and challenges. *Cancer Letters,* 2013, vol. 340, 284-295
- **DIETEL M ; JÖHRENS K ; LAFFERT MV ; HUMMEL M ; BLÄKER H ; PFITZNER BM ; LEHMANN A ; DENKERT C ; DARB-ESFAHANI S ; LENZE D.** A 2015 update on predictive molecular pathology and its role in targeted cancer therapy: a review focussing on clinical relevance. *Cancer Gene Therapy,* 2015, vol. 22, 417-430

- **SIKKEMA-RADDATZ B ; JOHANSSON LF ; DE BOER EN ; ALMOMANI R ; BOVEN LG ; VAN DEN BERG MP ; VAN SPAENDONCK-ZWARTS KY ; VAN TINTELEN JP ; SIJMONS RH ; JONGBLOED JDH.** Targeted Next-Generation Sequencing can Replace Sanger Sequencing in Clinical Diagnostics. *Human Mutation,* 2013, vol. 34, 1035-1042
- **SHAO D ; LIN Y ; LIU J ; WAN L ; LIU Z ; CHENG S ; FEI L ; DENG R ; WANG J ; CHEN X.** A targeted next-generation sequencing method for identifying clinically relevant mutation profiles in lung adenocarcinoma. *Scientific Reports,* 2016, vol. 6, 22338
- **DEVARAJAN B ; PRAKASH L ; KANNAN TR ; ABRAHAM AA ; KIM U ; MUTHUKKARUPPAN V ; VANNIARAJAN A.** Targeted next generation sequencing of RB1 gene for the molecular diagnosis of Retinoblastoma. *BMC Cancer,* 2015, vol. 15, http://www.biomedcentral.com/1471-2407/15/320
- **BOONHAM N ; KREUZE J ; WINTER S ; VAN DER VLUGT R ; BERGERVOET J ; TOMLINSON J ; MUMFORD R.** Methods in virus diagnostics: From ELISA to next generation sequencing. *Virus Research,* 2014, vol. 186, 20-31
- **PAK TR ; KASARSKIS A.** How Next-Generation Sequencing and Multiscale Data Analysis Will Transform Infectious Disease Management. *Clinical Infectious Diseases,* 2015, civ670
- **BARZON L ; LAVEZZO E ; MILITELLO V ; TOPPO S ; PALÙ G.** Applications of Next-Generation Sequencing Technologies to Diagnostic Virology. *International Journal of Molecular Sciences,* 2011, vol. 12, 7861-7884
- **SHEN R ; SESHAN VE.** FACETS: allele-specific copy number and clonal heterogeneity analysis tool for high-throughput DNA sequencing. *Nucleic Acids Research,* 2016, gkw520
- **JOHANSSON LF ; VAN DIJK F ; DE BOER EN ; VAN DIJK-BOS KK ; JONGBLOED JDH ; VAN DER HOUT AH ; WESTERS H ; SINKE RJ ; SWERTZ MA ; SIJMONS RH.** CoNVaDING: Single Exon Variation Detection in Targeted NGS Data. *Human Mutation,* 2016, vol. 37, 457-464
- **BOEVA V ; POPOVA T ; BLEAKLEY K ; CHICHE P ; CAPPO J ; SCHLEIERMACHER G ; JANOUEIX-LEROSEY I ; DELATTRE O ; BARILLOT E.** Control-FREEC: a tool for assessing copy number and allelic content using next-generation sequencing data. *Bioinformatics,* 2012, vol. 28, 423-425
- **DUAN J ; ZHANG J-G ; DENG H-W ; WANG Y-P.** CNV-TV: A robust method to discover copy number variation from short sequencing reads. *BMC Bioinformatics,* 2013, vol. 14, 150
- **ZHAO X ; WANG A ; WALTER V ; PATEL NM ; EBERHARD DA ; HAYWARD MC ; SALAZAR AH ; JO H ; SOLOWAY MG ; WILKERSON MD.** Combined Targeted DNA Sequencing in Non-Small Cell Lung Cancer (NSCLC) Using UNCseq and NGScopy, and RNA Sequencing Using UNCqeR for the Detection of Genetic Aberrations in NSCLC. Calogero RA, editor. *PLOS ONE,* 2015, vol. 10, e0129280
- **WINCHESTER L ; YAU C ; RAGOUSSIS J.** Comparing CNV detection methods for SNP arrays. *Briefings in Functional Genomics and Proteomics,* 2009, vol. 8, 353-366
- **ZHANG X ; DU R ; LI S ; ZHANG F ; JIN L ; WANG H.** Evaluation of copy number variation detection for a SNP array platform. *BMC bioinformatics,* 2014, vol. 15, 1
- **ZHANG D ; QIAN Y ; AKULA N ; ALLIEY-RODRIGUEZ N ; TANG J ; GERSHON ES ; LIU C.** Accuracy of CNV detection from GWAS data. *PLoS One,* 2011, vol. 6, e14511
- **BARREAU O ; DE REYNIES A ; WILMOT-ROUSSEL H ; GUILLAUD-BATAILLE M ; AUZAN C ; RENÉ-CORAIL F ; TISSIER F ; DOUSSET B ; BERTAGNA X ; BERTHERAT J.** Clinical and Pathophysiological Implications of Chromosomal Alterations in Adrenocortical Tumors: An Integrated Genomic Approach. *The Journal of Clinical Endocrinology & Metabolism,* 2012, vol. 97, E301-E311
- **ASSIÉ G ; LETOUZÉ E ; FASSNACHT M ; JOUINOT A ; LUSCAP W ; BARREAU O ; OMEIRI H ; RODRIGUEZ S ; PERLEMOINE K ; RENÉ-CORAIL F.** Integrated genomic characterization of adrenocortical carcinoma. *Nature Genetics,* 2014, vol. 46, 607-612
- **POPOVA T ; MANIÉ E ; STOPPA-LYONNET D ; RIGAILL G ; BARILLOT E ; STERN MH.** Genome Alteration Print (GAP): a tool to visualize and mine complex cancer genomic profiles obtained by SNP arrays. *Genome biology,* 2009, vol. 10, 1
- **LI L-C ; DAHIYA R.** MethPrimer: designing primers for methylation PCRs. *Bioinformatics,* 2002, 1427-1431
- **KRUEGER F ; ANDREWS SR.** Bismark: a flexible aligner and methylation caller for Bisulfite-Seq applications. *Bioinformatics,* 2011, vol. 27, 1571-1572
- **TARIQ K ; GHIAS K.** Colorectal cancer carcinogenesis: a review of mechanisms. *Cancer biology & medicine,* 2016, vol. 13, 120
- **ASSIÉ G ; LAFRAMBOISE T ; PLATZER P ; BERTHERAT J ; STRATAKIS CA ; ENG C.** SNP arrays in heterogeneous tissue: highly accurate collection of both germline and somatic genetic information from unpaired single tumor samples. *Am J Hum Genet,* 2008, vol. 82, 903-915

- **LANGMEAD B ; SALZBERG SL.** Fast gapped-read alignment with Bowtie 2. *Nat Methods,* 2012, vol. 9, 357-359
- **HARRISON CJ.** Targeting signaling pathways in acute lymphoblastic leukemia: new insights. *Hematology Am Soc Hematol Educ Program,* 2013, vol. 2013, 118-125
- **MOALIC-ALLAIN V ; MERCIER B ; GUEGUEN P ; FEREC C.** Next generation sequencing with a semi-conductor technology (Ion Torrent PGM™) for HLA typing: overall workflow performance and debate. *Ann Biol Clin,* 2016, vol. 74, 449-456
- **WITTE T ; PLASS C ; GERHAUSER C.** Pan-cancer patterns of DNA methylation. *Genome Med,* 2014, vol. 6, 66
- **JEONG HM ; LEE S ; CHAE H ; KIM R ; KWON MJ ; OH E ; CHOI Y-L ; KIM S ; SHIN YK.** Efficiency of methylated DNA immunoprecipitation bisulphite sequencing for whole-genome DNA methylation analysis. *Epigenomics,* 2016, vol. 8, 1061-1077
- **GICQUEL C ; BERTAGNA X ; GASTON V ; COSTE J ; LOUVEL A ; BAUDIN E ; BERTHERAT J ; CHAPUIS Y ; DUCLOS JM ; SCHLUMBERGER M.** Molecular markers and long-term recurrences in a large cohort of patients with sporadic adrenocortical tumors. *Cancer Res.,* 2001, vol. 61 (18), 6762-6767
- **LE TOURNEAU C ; DELORD J-P ; GONÇALVES A ; GAVOILLE C ; DUBOT C ; ISAMBERT N ; CAMPONE M ; TRÉDAN O ; MASSIANI M-A ; MAUBORGNE C.** SHIVA investigators: Molecularly targeted therapy based on tumour molecular profiling versus conventional therapy for advanced cancer (SHIVA): a multicentre, open-label, proof-of-concept, randomised, controlled phase 2 trial. *Lancet Oncol,* 2015, vol. 16, 1324-1334
- **JOUINOT A ; ASSIE G ; LIBE R ; FASSNACHT M ; PAPATHOMAS T ; BARREAU O ; DE LA VILLEON B ; FAILLOT S ; HAMZAOUI N ; NEOU M.** DNA Methylation Is an Independent Prognostic Marker of Survival in Adrenocortical Cancer. *J Clin Endocrinol Metab,* 2017, vol. 102, 923-932

SEQUENCE LISTING

<110> INSERM
APHP
CNRS
UNIVERSITE PARIS DESCARTES
ASSIE, Guillaume
LETOURNEUR, Franck
BERTHERAT, Jérôme

<120> METHOD FOR MOLECULAR TYPING OF TUMORS IN A SINGLE TARGETED NEXT GENERATION SEQUENCING EXPERIMENT

<130> BNT221709EP00

<160> 30

<170> PatentIn version 3.5

<210> 1
<211> 28
<212> DNA
<213> Artificial Sequence

<220>
<223> Primer

<400> 1
ggagtgttga attttagagg aagaaatt 28

<210> 2
<211> 30
<212> DNA
<213> Artificial Sequence

<220>
<223> Primer

<400> 2
ccaaaacaac cattcttcaa actaactata 30

<210> 3
<211> 27
<212> DNA
<213> Artificial Sequence

<220>
<223> Primer

<400> 3
taggtttaag gagatgtaaa tggggga 27

<210> 4
<211> 24
<212> DNA
<213> Artificial Sequence

<220>
<223> Primer

<400> 4
ccctaaaccc ttccaaccaa aatc 24

<210> 5
<211> 22
<212> DNA
<213> Artificial Sequence

<220>
<223> Primer

<400> 5
gggggtttag aagggatttt tt 22

<210> 6
<211> 23
<212> DNA
<213> Artificial Sequence

<220>
<223> Primer

<400> 6
ccacctctat tcctactcca ccc 23

<210> 7
<211> 22
<212> DNA
<213> Artificial Sequence

<220>
<223> Primer

<400> 7
ggggtttgga gaagagtagg aa 22

<210> 8
<211> 25
<212> DNA
<213> Artificial Sequence

<220>
<223> Primer

<400> 8
cccaacccca tctctccaac ctatc 25

<210> 9
<211> 25
<212> DNA
<213> Artificial Sequence

<220>
<223> Primer

<400> 9
aagtgtttgg ggaaggttgg ttgtt 25

<210> 10
<211> 27
<212> DNA
<213> Artificial Sequence

<220>
<223> Primer

<400> 10
ctaaacaacc tcctaatcat cctatcc 27

<210> 11
<211> 27
<212> DNA
<213> Artificial Sequence

<220>
<223> Primer

<400> 11
tttagttgtt taatttgaaa gaggggt 27

<210> 12
<211> 26
<212> DNA
<213> Artificial Sequence

<220>
<223> Primer

<400> 12
aaaaattata cacccccaaa aaaaac 26

<210> 13
<211> 24
<212> DNA
<213> Artificial Sequence

<220>
<223> Primer

<400> 13
ggagagagga agggttaagg agta 24

<210> 14
<211> 26
<212> DNA
<213> Artificial Sequence

<220>
<223> Primer

<400> 14
caatcacttt ccccaacacc aaattc 26

<210> 15
<211> 26

<212>  DNA
<213>  Artificial Sequence

<220>
<223>  Primer

<400>  15
gtgaattagg tttggaaggg gatata                                          26

<210>  16
<211>  31
<212>  DNA
<213>  Artificial Sequence

<220>
<223>  Primer

<400>  16
ccccaaaact aactaataaa aaaatttaac a                                    31

<210>  17
<211>  27
<212>  DNA
<213>  Artificial Sequence

<220>
<223>  Primer

<400>  17
ttggggtaaa agtgatattg tttaggt                                         27

<210>  18
<211>  27
<212>  DNA
<213>  Artificial Sequence

<220>
<223>  Primer

<400>  18
ctacccaaat atcccctaaa actcttc                                         27

<210>  19
<211>  25
<212>  DNA
<213>  Artificial Sequence

<220>
<223>  Primer

<400>  19
tttgttgtat gtttttttgg gattt                                           25

<210>  20
<211>  25
<212>  DNA
<213>  Artificial Sequence

<220>
<223> Primer

<400> 20
aaccttccaa caaccaaaat ttaaa 25

<210> 21
<211> 23
<212> DNA
<213> Artificial Sequence

<220>
<223> Primer

<400> 21
tatggaattt ttttggttgg aga 23

<210> 22
<211> 27
<212> DNA
<213> Artificial Sequence

<220>
<223> Primer

<400> 22
tacccaaaaa accaataaat aaaaaac 27

<210> 23
<211> 26
<212> DNA
<213> Artificial Sequence

<220>
<223> Primer

<400> 23
gtttttaggt ttttagttgg gttgtt 26

<210> 24
<211> 27
<212> DNA
<213> Artificial Sequence

<220>
<223> Primer

<400> 24
ctctaaacta aactcctcat ctacccc 27

<210> 25
<211> 26
<212> DNA
<213> Artificial Sequence

<220>
<223> Primer

<400> 25
tttggtttag taggttagtg ggtagg 26

<210> 26
<211> 29
<212> DNA
<213> Artificial Sequence

<220>
<223> Primer

<400> 26
aaaaaaacaa aaaataaaac taaaaaacc 29

<210> 27
<211> 26
<212> DNA
<213> Artificial Sequence

<220>
<223> Primer

<400> 27
gaagggggta gagagagtta tgattt 26

<210> 28
<211> 28
<212> DNA
<213> Artificial Sequence

<220>
<223> Primer

<400> 28
caataaaaaa aatccctaca aaaacaac 28

<210> 29
<211> 24
<212> DNA
<213> Artificial Sequence

<220>
<223> Primer

<400> 29
gaggggtaga gattttgttt ttga 24

<210> 30
<211> 27
<212> DNA
<213> Artificial Sequence

<220>
<223> Primer

<400> 30
aaaattaaat aaattcccca tatcacc 27